# EUROPEAN PATENT APPLICATION

(11) **EP 3 950 060 A1**
(43) Date of publication of application: **09.02.2022**
(21) Application number: 20784669.2
(22) Date of filing: 03.04.2020
(51) Int. Cl.: A61P 25/00, A61P 25/14, A61P 25/16, A61P 25/28, C07C 39/14, C07C 47/57, C07C 235/66, C12N 5/0775, C12N 1/00, A61K 31/05, A61K 31/11, A61K 31/165, A61K 31/4025, A61K 31/404

(54) **COMPOSITION FOR PROMOTING SECRETION OF EXTRACELLULAR VESICLES**

(30) Priority: 04.04.2019 JP 2019071759
(71) Applicant: Nissan Chemical Corporation, Tokyo 103-6119 (JP); National University Corporation Kanazawa University, Ishikawa 920-1192 (JP)
(72) Inventor: MATOBA Kazutaka, Shiraoka-shi, Saitama 349-0294 (JP); KIDA Katsuhiko, Shiraoka-shi, Saitama 349-0294 (JP); NISHINO Taito, Shiraoka-shi, Saitama 349-0294 (JP); HANAYAMA Rikinari, Kanazawa-shi, Ishikawa 920-1192 (JP); YOSHIDA Takeshi, Kanazawa-shi, Ishikawa 920-1192 (JP)
(74) Representative: dompatent von Kreisler Selting Werner - Partnerschaft von Patent- und Rechtsanwälten mbB
(86) International application number: PCT/JP2020/015310
(87) International publication number: WO 2020/204161

(57) **Abstract**

This is to provide a composition for promoting secretion of extracellular vesicles.

Provided is a composition for promoting secretion of extracellular vesicles, which comprises a compound having a structure of the formula I (wherein each substituent is as defined in the specification), a racemate thereof or a stereoisomer thereof, or a salt thereof, or a compound having a structure of the formula II (wherein each substituent is as defined in the specification), or a stereoisomer thereof, or a salt thereof, or, cucurbitacin, or a stereoisomer thereof, or a salt thereof.

## Description

### TECHNICAL FIELD

The present invention relates to a composition for promoting secretion of extracellular vesicles from cells.

### BACKGROUND ART

Extracellular vesicles are vesicles secreted by cells whose membrane structure consists of a lipid bilayer, similar to the cells themselves or organelles in the cells, and it has been known that they are stably present in all body fluids such as saliva, blood, urine, amniotic fluid, etc., and cell culture fluid.

As extracellular vesicles, for example, Exosomes, Microvesicles (MV), Apoptotic Bodies, etc., have been known. Exosomes are vesicles of about 20 to about 200 nm derived from endocytosis pathways, and as the constitutional components, proteins, nucleic acids (mRNA, miRNA, non-coding RNA, etc.), etc., have been known, and they can have a function of controlling intercellular communication. Microvesicles (MV) are vesicles of about 50 to about 1,000 nm derived from cytoplasmic membrane, and as the constitutional components, proteins, nucleic acids (mRNA, miRNA, non-coding RNA, etc.), etc., have been known, and they can have a function of controlling intercellular communication. Apoptotic bodies are vesicles of about 500 to about 2,000 nm derived from cytoplasmic membrane, and as the constitutional components, fragmented nuclei, cell organ (organelles), etc., have been known, and they can have a function of inducing phagocytosis, etc.

In recent years, it has been reported that extracellular vesicles are secreted from various cells (for example, mesenchymal stem cells (MSC), etc.), and it has been attracted attention that they function as mediators of intercellular communication in the living body, and the relationship with diseases such as cancer and neurodegenerative diseases. For example, in Non-Patent Document 1, it has been suggested that the function of removing amyloid β protein by microglia is promoted by secretion of exosomes. Also, in Non-Patent Document 2, it has been suggested that exosomes are involved in metabolism of amyloid β in the brain.

Extracellular vesicles secreted by mesenchymal stem cells (MSC) have been investigated for applying to many diseases such as cancer, heart diseases, neurodegenerative diseases and immune system diseases, due to their multifaced functions such as anti-inflammatory action and cytoprotective action. For example, in Patent Document 1, it has been described that exosomes derived from mesenchymal stem cells can be used to treat diseases such as heart failure. Also, in Patent Document 2, it has been described that extracellular vesicles derived from mesenchymal stem cells can be used for preventing and/or treating various corneal epithelial diseases.

From such a background, in recent years, research has been progressed about the substances that promote secretion of extracellular vesicles. For example, in Patent Document 3, it has been reported a method for promoting formation of exosomes from stem cells by culturing the stem cells in a medium containing thrombin. Also, in Patent Document 4, it has been reported that ceramide promotes production of exosomes.

### PRIOR ART DOCUMENTS

### PATENT DOCUMENTS

Patent Document 1: JP 2011-513217A
Patent Document 2: WO 2017/022809
Patent Document 3: WO 2015/088288
Patent Document 4: JP 2018-150290A

### NON-PATENT DOCUMENTS

Non-Patent Document 1: Yuyama K et al., J Biol Chem., 289 (35), 24488-98 (2014)
Non-Patent Document 2: Yuyama K et al., J Biol Chem., 287 (14), 10977-89 (2012)

### SUMMARY OF THE INVENTION

### PROBLEMS TO BE SOLVED BY THE INVENTION

However, with regard to the substances which promote secretion of extracellular vesicles, a number of the reports is limited and development of a novel composition for promoting secretion of extracellular vesicles has been desired.

Accordingly, an object of the present invention is to provide a composition for promoting secretion of extracellular vesicles.

### MEANS FOR SOLVING THE PROBLEMS

In view of the above-mentioned problems, the present inventors have earnestly studied, and as a result, they have found that a composition containing a specific compound unexpectedly promotes secretion of extracellular vesicles, whereby the present invention has been completed.

Accordingly, the present invention provides the following, in summary.
[1] A composition for promoting secretion of extracellular vesicles, which comprises a compound having a structure represented by the formula I: 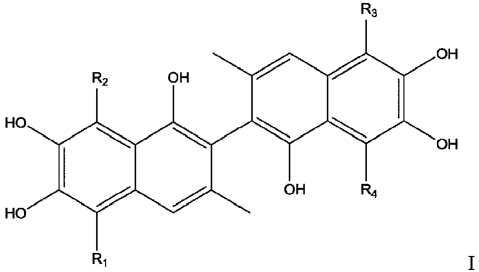 wherein
   R₁ is C₁-C₆ alkyl which may be substituted by hydroxy, halogen, amino, nitro or cyano, or -C(O)-R₇,
   R₂ is hydrogen, hydroxy, C₁-C₆ alkyl or -C(O)-R₅,
   R₃ is C₁-C₆ alkyl which may be substituted by hydroxy, halogen, amino, nitro or cyano, or -C(O)-R₈,
   R₄ is hydrogen, hydroxy, C₁-C₆ alkyl or -C(O)-R₆,
   R₅ and R₆ are independently hydrogen; hydroxy; C₁-C₆ alkyl which may be substituted by hydroxy, halogen, amino, nitro or cyano; or C₁-C₆ alkoxy which may be substituted by hydroxy, halogen, amino, nitro or cyano,
   R₇ and R₈ are independently hydrogen, hydroxy or -NR₉R₁₀,
   R₉ and R₁₀ are independently hydrogen, or C₁-C₆ alkyl which may be substituted by hydroxy, halogen, amino, nitro, cyano or phenyl,
      a racemate thereof or a stereoisomer thereof, or a salt thereof.
[2] A composition for promoting secretion of extracellular vesicles, which comprises a compound having a structure of the formula II:

   A-B II

   wherein
   A is 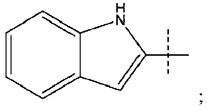 or 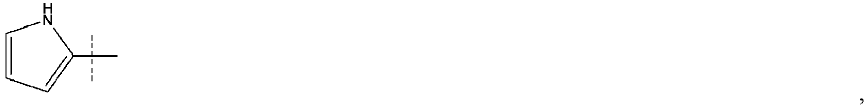
   B is 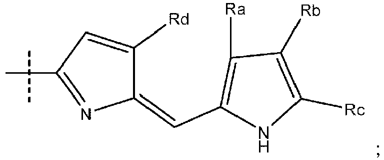 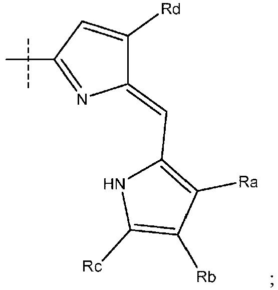 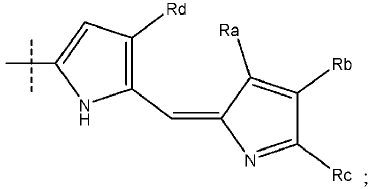 or 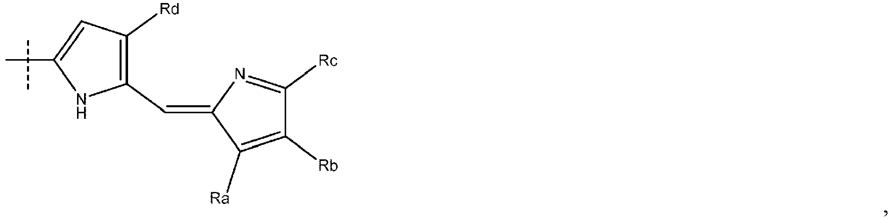 in these formulae,
      each Ra to Rc is independently hydrogen, hydroxy, halogen, or C₁-C₆ alkyl which may be substituted by C₁-C₆ alkyl, hydroxy, halogen, amino, nitro or cyano,
      each Rd is independently hydrogen, hydroxy, C₁-C₆ alkyl, or C₁-C₆ alkoxy which may be substituted by hydroxy, halogen, amino, nitro or cyano,
      or a stereoisomer thereof, or a salt thereof.
[3] A composition for promoting secretion of extracellular vesicles, which comprises cucurbitacin, or a stereoisomer thereof, or a salt thereof.
[4] The composition described in [1], wherein R₁ is C₁-C₆ alkyl and R₃ is C₁-C₆ alkyl.
[5] The composition described in [1] or [4], wherein R₂ is -C(O)-R₅ and R₃ is -C(O)-R₆.
[6] The composition described in [5], wherein R₅ is hydrogen and R₆ is hydrogen.
[7] The composition described in [1], wherein the compound having a structure of the formula I is selected from the group consisting of the following: 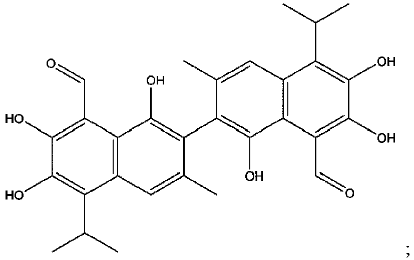 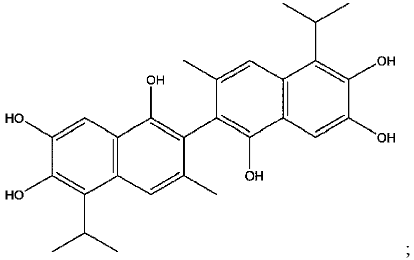 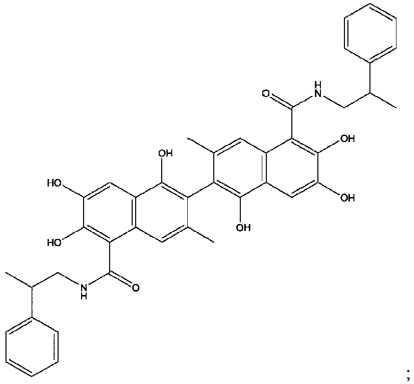
[8] The composition described in [1], wherein the compound having a structure of the formula I is selected from the group consisting of (±)-gossypol, (S)-gossypol, (+)-apogossypol, (R)-(-)-gossypol and sabutoclax.
[9] The composition described in [2], wherein A is the following: 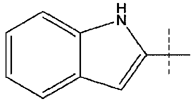
[10] The composition described in [2] or [9], wherein B is the following: 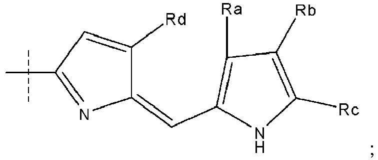 or 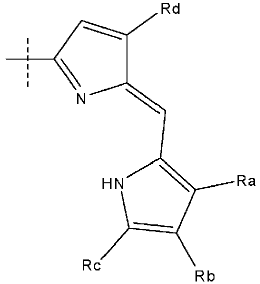
[11] The composition described in any of [2], [9] and [10], wherein Rd is C₁-C₆ alkoxy.
[12] The composition described in [2], wherein the compound having a structure of the formula II is selected from the group consisting of the following: 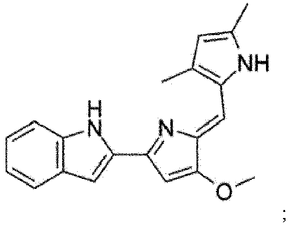 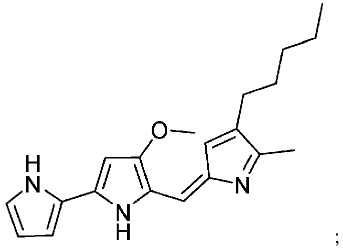 and 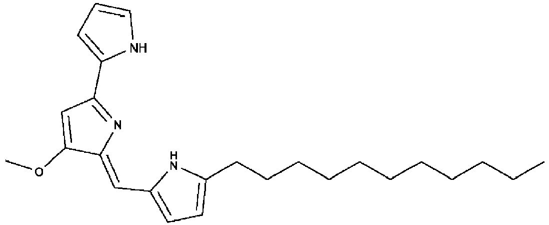
[13] The composition described in [2], wherein the compound having a structure of the formula II is selected from the group consisting of obatoclax, prodigiosin and undecylprodigiosin.
[14] The composition described in [3], wherein cucurbitacin is selected from the group consisting of cucurbitacin A, cucurbitacin B, cucurbitacin C, cucurbitacin D, cucurbitacin E, cucurbitacin F, cucurbitacin G, cucurbitacin H, cucurbitacin I, cucurbitacin J, cucurbitacin K, cucurbitacin L, cucurbitacin M, cucurbitacin N, cucurbitacin O, cucurbitacin P, cucurbitacin Q, cucurbitacin R, cucurbitacin S and cucurbitacin T.
[15] The composition described in [3] or [14], wherein cucurbitacin is cucurbitacin B.
[16] The composition described in any of [1] to [15], which is for promoting secretion of extracellular vesicles from cells derived from adipose tissues.
[17] The composition described in any of [1] to [16], which is for promoting secretion of extracellular vesicles from mesenchymal stem cells.
[18] The composition described in any of [1] to [17], which is a medium composition.
[19] A method for promoting secretion of extracellular vesicles from cells *in vitro* or *ex vivo,* which comprises using the composition described in [18].
[20] A culture supernatant obtainable from the method described in [19].
[21] Extracellular vesicles obtainable from the culture supernatant described in [20].
[22] A method for producing extracellular vesicles *in vitro* or *ex vivo,* which comprises using the composition described in any of [1] to [18].
[23] The method described in [22], which contains a step of bringing the composition described in any of [1] to [18] into contact with cells.

### EFFECT OF THE INVENTION

According to the present invention, a composition for promoting secretion of extracellular vesicles can be provided.

### BRIEF DESCRIPTION OF THE DRAWINGS

Fig. 1A shows the results of evaluating secreted amounts of extracellular vesicles when the compound of Example 1 or 5 was added to U-87 MG cells by the Tim4-CD9 ELISA method, and shown as a relative secreted amount to the secreted amount of extracellular vesicles when the solvent alone (control) was added in Test Example 1.
Fig. 1B shows the results of evaluating secreted amounts of extracellular vesicles when each of the compounds of Examples 1 and 5 was added to U-87 MG cells by the Tim4-CD63 ELISA method, and shown as a relative secreted amount to the secreted amount of extracellular vesicles when the solvent alone (control) was added in Test Example 1.
Fig. 2 shows the results of evaluating cytotoxicity when each of the compounds of Examples 1 and 5 was added to U-87 MG cells with the LDH amount in the culture supernatant in Test Example 2.
Fig. 3A shows the results of evaluating the secreted amount of extracellular vesicles when each of the compounds of Examples 1 and 5 was added to mesenchymal stem cells derived from human adipose tissue by the Tim4-CD63 ELISA method in Test Example 3.
Fig. 3B shows the results of evaluating the secreted amount of extracellular vesicles when each of the compounds of Examples 1 to 4 and 6 to 8 was added to mesenchymal stem cells derived from human adipose tissue by the Tim4-CD63 ELISA method in Test Example 3.
Fig. 4A shows the results of evaluating cytotoxicity when each of the compounds of Examples 1 and 5 was added to mesenchymal stem cells derived from human adipose tissue with the LDH amount in the culture supernatant in Test Example 4.
Fig. 4B shows the results of evaluating cytotoxicity when each of the compounds of Examples 1 to 4 and 6 to 8 was added to mesenchymal stem cells derived from human adipose tissue with the LDH amount in the culture supernatant in Test Example 4.
Fig. 5A shows the results of evaluating cell proliferation activity (cell viability) when each of the compounds of Examples 1 and 5 was added to mesenchymal stem cells derived from human adipose tissue in Test Example 5.
Fig. 5B shows the results of evaluating cell proliferation activity (cell viability) when each of the compounds of Examples 1 to 4 and 6 to 8 was added to mesenchymal stem cells derived from human adipose tissue in Test Example 5.
Fig. 6 shows the results of evaluating the number of living cells when exosomes obtained by treating each of the compounds of Examples 2 and 5 with mesenchymal stem cells derived from human adipose tissue were added to H9C2 cells under hypoxic treatment in Test Example 6.
Fig. 7A shows the results of evaluating the secreted amount of extracellular vesicles when each of the compounds of Examples 1 to 8 was added to mesenchymal stem cells derived from human bone marrow by the Tim4-CD63 ELISA method in Test Example 7.
Fig. 7B shows the results of evaluating the secreted amount of extracellular vesicles when each of the compounds of Examples 1 to 8 was added to mesenchymal stem cells derived from human cord matrix by the Tim4-CD63 ELISA method in Test Example 7.
Fig. 7C shows the results of evaluating cytotoxicity when each of the compounds of Examples 1 to 8 was added to mesenchymal stem cells derived from human bone marrow with the LDH amount in the culture supernatant in Test Example 8.
Fig. 7D shows the results of evaluating cytotoxicity when each of the compounds of Examples 1 to 8 was added to mesenchymal stem cells derived from human umbilical cord matrix with the LDH amount in the culture supernatant in Test Example 8.
Fig. 7E shows the results of evaluating cell proliferation activity (cell viability) when each of the compounds of Examples 1 to 8 was added to mesenchymal stem cells derived from human bone marrow in Test Example 9.
Fig. 7F shows the results of evaluating cell proliferation activity (cell viability) when each of the compounds of Examples 1 to 8 was added to mesenchymal stem cells derived from human umbilical cord matrix in Test Example 9.
Fig. 8A shows the results of evaluating the secreted amount of extracellular vesicles when each of the compounds of Examples 1, 5 and 9 was added to 293 cells by the Tim4-CD63 ELISA method in Test Example 10.
Fig. 8B shows the results of evaluating the secreted amount of extracellular vesicles when each of the compounds of Examples 1, 5 and 9 was added to 293T cells by the Tim4-CD63 ELISA method in Test Example 10.
Fig. 8C shows the results of evaluating the secreted amount of extracellular vesicles when each of the compounds of Examples 1, 5 and 9 was added to 293 cells by the Tim4-CD81 ELISA method in Test Example 10.
Fig. 8D shows the results of evaluating the secreted amount of extracellular vesicles when each of the compounds of Examples 1, 5 and 9 was added to 293T cells by the Tim4-CD81 ELISA method in Test Example 10.
Fig. 8E shows the results of evaluating the secreted amount of extracellular vesicles when each of the compounds of Examples 1, 5 and 9 was added to 293 cells by the nanoparticle tracking analysis system in Test Example 11.
Fig. 8F shows the results of evaluating the secreted amount of extracellular vesicles when each of the compounds of Examples 1, 5 and 9 was added to 293T cells by the nanoparticle tracking analysis system in Test Example 11.
Fig. 8G shows the results of evaluating cytotoxicity when each of the compounds of Examples 1, 5 and 9 was added to 293 cells with the LDH amount in the culture supernatant in Test Example 12.
Fig. 8H shows the results of evaluating cytotoxicity when each of the compounds of Examples 1, 5 and 9 was added to 293T cells with the LDH amount in the culture supernatant in Test Example 12.
Fig. 81 shows the results of evaluating cell proliferation activity when each of the compounds of Examples 1, 5 and 9 was added to 293 cells in Test Example 13.

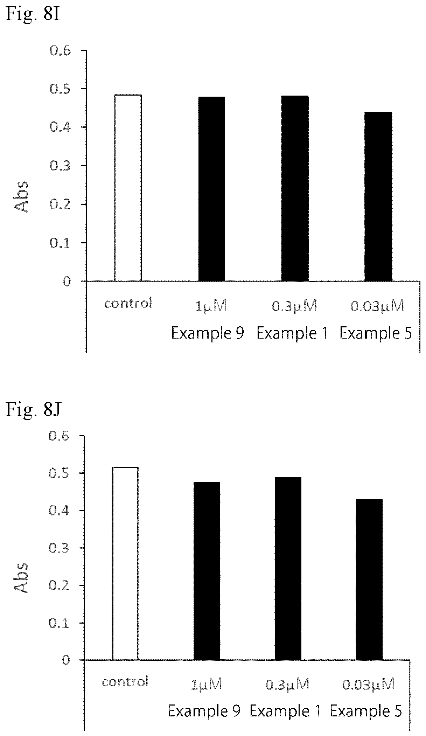

Fig. 8J shows the results of evaluating cell proliferation activity when each of the compounds of Examples 1, 5 and 9 was added to 293 cells in Test Example 13.

### EMBODIMENT TO CARRY OUT THE INVENTION

The present invention provides a composition for promoting secretion of extracellular vesicles, which comprises a compound having a structure of the formula I: 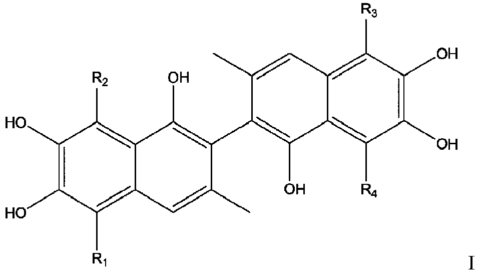 [wherein
R₁ is C₁-C₆ alkyl which may be substituted by hydroxy, halogen, amino, nitro or cyano, or -C(O)-R₇,
R₂ is hydrogen, hydroxy, C₁-C₆ alkyl or -C(O)-R₈,
R₃ is C₁-C₆ alkyl which may be substituted by hydroxy, halogen, amino, nitro or cyano, or -C(O)-R₈,
R₄ is hydrogen, hydroxy, C₁-C₆ alkyl or -C(O)-R₆,
R₅ and R₆ are independently hydrogen; hydroxy; C₁-C₆ alkyl which may be substituted by hydroxy, halogen, amino, nitro or cyano; or C₁-C₆ alkoxy which may be substituted by hydroxy, halogen, amino, nitro or cyano,
R₇ and R₈ are independently hydrogen, hydroxy or -NR₉R₁₀,
R₉ and R₁₀ are independently hydrogen, or C₁-C₆ alkyl which may be substituted by hydroxy, halogen, amino, nitro, cyano or phenyl], a racemate thereof or a stereoisomer thereof, or a salt thereof or a compound having a structure of the formula II:

   A-B II

   [wherein
   A is 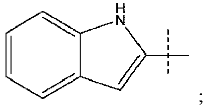 or 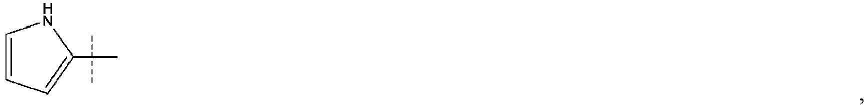
   B is 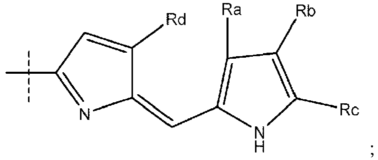 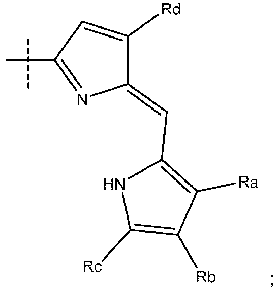 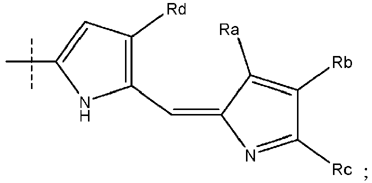 or 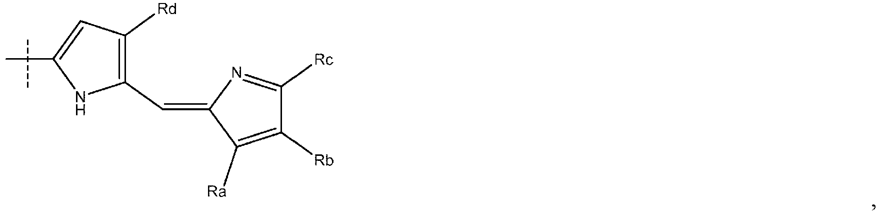 in these formulae,
      each Ra to Rc is independently hydrogen, hydroxy, halogen, or C₁-C₆ alkyl which may be substituted by C₁-C₆ alkyl, hydroxy, halogen, amino, nitro or cyano,
      each Rd are independently hydrogen, hydroxy, C₁-C₆ alkyl, or C₁-C₆ alkoxy which may be substituted by hydroxy, halogen, amino, nitro or cyano],
      or a stereoisomer thereof, or a salt thereof,
      or cucurbitacin, or a stereoisomer thereof, or a salt thereof.

The present invention also provides a compound having a structure of the formula I, a racemate thereof or a stereoisomer thereof, or a salt thereof, or a compound having a structure of the formula II, or a stereoisomer thereof, or a salt thereof, or cucurbitacin, or a stereoisomer thereof, or a salt thereof, for promoting secretion of extracellular vesicles.

The present invention also provides a use for producing a composition for promoting secretion of extracellular vesicles, which comprises a use of a compound having a structure of the formula I, a racemate thereof or a stereoisomer thereof, or a salt thereof, or a compound having a structure of the formula II, or a stereoisomer thereof, or a salt thereof, or cucurbitacin, or a stereoisomer thereof, or a salt thereof.

The present invention also provides a method for promoting secretion of extracellular vesicles, which method comprises administering a compound having a structure of the formula I, a racemate thereof or a stereoisomer thereof, or a salt thereof, or a compound having a structure of the formula II, or a stereoisomer thereof, or a salt thereof, or cucurbitacin, or a stereoisomer thereof, or a salt thereof, to a subject who requires it.

The term "hydroxy" as used in the present specification means a group represented by the formula "-OH".

The term "halogen" as used in the present specification means, but is not limited thereto, for example, fluorine, chlorine, bromine, iodine and the like.

The term "amino" as used in the present specification means a group represented by the formula "-NH₂".

The term "nitro" as used in the present specification means a group represented by the formula "-NO₂".

The term "cyano" as used in the present specification means a group represented by the formula "-CN".

The terms "C₁-C₆ alkyl" as used in the present specification mean a saturated linear or branched hydrocarbon group containing 1 to 6 carbon atoms (which are not limited to these, and it contains, for example, methyl, ethyl, propyl, isopropyl, n-butyl, i-butyl, t-butyl, pentyl, hexyl, etc.). Preferable C₁-C₆ alkyl is C₁₋₄ alkyl (for example, it contains methyl, ethyl, propyl, butyl, isopropyl, etc.), more preferably methyl and isopropyl.

The terms "C₁-C₆ alkyl which may be substituted by hydroxy, halogen, amino, nitro or cyano" as used in the present specification mean a group in which one or more hydrogen atoms of the C₁-C₆ alkyl may be substituted by hydroxy, halogen, amino, nitro or cyano, or may not be substituted.

The terms "C₁-C₆ alkoxy" as used in the present specification mean a group represented by the formula "-O-C₁-C₆ alkyl". The C₁-C₆ alkoxy may be mentioned, which are not limited to these, for example, methoxy, ethoxy, propoxy, isopropoxy, butoxy, t-butoxy, etc., preferably methoxy.

The terms "C₁-C₆ alkoxy which may be substituted by hydroxy, halogen, amino, nitro or cyano" as used in the present specification mean C₁-C₆ alkoxy may be substituted by hydroxy, halogen, amino, nitro or cyano, or may not be substituted.

The terms "C₁-C₆ alkyl which may be substituted by hydroxy, halogen, amino, nitro, cyano or phenyl" as used in the present specification mean a group in which one or more hydrogen atoms of the C₁-C₆ alkyl may be substituted by hydroxy, halogen, amino, nitro, cyano or phenyl, or may not be substituted.

The terms "C₁-C₆ alkyl which may be substituted by C₁-C₆ alkyl, hydroxy, halogen, amino, nitro or cyano" as used in the present specification mean a group in which one or more hydrogen atoms of the C₁-C₆ alkyl may be substituted by C₁-C₆ alkyl, hydroxy, halogen, amino, nitro or cyano, or may not be substituted.

The broken line that intersects the bond, shown in the chemical structural formula in the present specification, indicates the point of bond to the remainder of the molecule or the remainder of the fragment of the molecule of the atom to which the broken line is connected in the chemical structure.

The terms "racemate" as used in the present specification means an equimolar mixture of two enantiomers and a material having no optical activity.

In the terms "stereoisomer" as used in the present specification, enantiomers, diastereomers, etc., are included.

The term "salt" as used in the present specification may be mentioned, which is not limited to these, for example, an acid addition salt with an inorganic acid or an organic acid, a salt with a metal, a salt with an organic base, etc. The "salt" used in the present specification is preferably a pharmaceutically acceptable salt.

The terms "pharmaceutically acceptable salt" as used in the present specification mean, which is not limited to these, there may be mentioned, for example, acid addition salts with inorganic acids (which are not limited to these, there may be mentioned, for example, hydrochloric acid, hydrobromic acid, sulfuric acid, nitric acid, carbonic acid, phosphoric acid, etc.) or organic acids (which are not limited to these, there may be mentioned, for example, formic acid, acetic acid, propionic acid, glycolic acid, gluconic acid, lactic acid, mesylic acid, pyruvic acid, oxalic acid, malic acid, maleic acid, malonic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, salicylic acid, etc.); salts with metals (which are not limited to these, there may be mentioned, for example, sodium, potassium, calcium, magnesium, iron, zinc, copper, manganese, etc.); ammonium salts; salts with organic bases (which are not limited to these, there may be mentioned, for example, isopropylamine, trimethylamine, diethylamine, triethylamine, tripropylamine, ethanolamine, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, etc.) , and the like.

As the "cucurbitacin" as used in the present specification, which are not limited to these, there may be mentioned, for example, cucurbitacin A, cucurbitacin B, cucurbitacin C, cucurbitacin D, cucurbitacin E, cucurbitacin F, cucurbitacin G, cucurbitacin H, cucurbitacin I, cucurbitacin J, cucurbitacin K, cucurbitacin L, cucurbitacin M, cucurbitacin N, cucurbitacin O, cucurbitacin P, cucurbitacin Q, cucurbitacin R, cucurbitacin S, cucurbitacin T, a derivative thereof (for example, a glycoside, a dihydro derivative, an oxo derivative, a deoxo derivative, etc.) and the like.

The term "glycoside" as used in the present specification refers to a compound formed by binding a hydroxyl group of a sugar to a non-carbohydrate compound. The sugar in the glycoside may be a monosaccharide, or may be a disaccharide or a plurality of sugars more than it. As the sugars constituting the glycoside, which are not limited to these, there may be mentioned, for example, aldoses such as glucose, mannose, galactose, fucose, rhamnose, arabinose, xylose, etc., ketoses such as fructose, etc., uronic acids such as glucuronic acid, galacturonic acid, mannuronic acid, etc., apiose, rutinose, etc. Also, the sugars constituting the glycoside may be a D-isomer, an L-isomer, or a mixture (DL-isomer) of the D-isomer and the L-isomer.

In the compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof contained in the composition described in the present specification, a solvate thereof (for example, a hydrate thereof), a crystal polymorphism thereof, a mixture of isomers, etc., are also included.

The term "solvate" as used in the present specification means an associate or a complex, etc., of one or more solvent molecules and the compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof. As the solvents, which are not limited to these, there may be mentioned, for example, water, methanol, ethanol, isopropanol, DMSO, acetic acid, ethyl acetate, etc.

In one embodiment of the present invention, it is provided a composition described in the present specification which contains the compound having a structure of the formula I, a racemate thereof or a stereoisomer thereof, or a salt thereof.

In one embodiment of the present invention, it is provided a composition (or a compound, use or method) described in the present specification, wherein R₁ is C₁-C₆ alkyl.

In one embodiment of the present invention, it is provided a composition (or a compound, use or method) described in the present specification, wherein R₃ is C₁-C₆ alkyl.

In one embodiment of the present invention, it is provided a composition (or a compound, use or method) described in the present specification, wherein R₁ is C₁-C₆ alkyl and R₃ is C₁-C₆ alkyl.

In a preferred embodiment of the present invention, it is provided a composition (or a compound, use or method) described in the present specification, wherein R₁ is isopropyl.

In a preferred embodiment of the present invention, it is provided a composition (or a compound, use or method) described in the present specification, wherein R₃ is isopropyl.

In a more preferred embodiment of the present invention, it is provided a composition (or a compound, use or method) described in the present specification, wherein R₁ is isopropyl and R₃ is isopropyl.

In one embodiment of the present invention, it is provided a composition (or a compound, use or method) described in the present specification, wherein R₂ is -C(O)-R₅.

In one embodiment of the present invention, it is provided a composition (or a compound, use or method) described in the present specification, wherein R₄ is -C(O)-R₆.

In a preferred embodiment of the present invention, it is provided a composition (or a compound, use or method) described in the present specification, wherein R₂ is -C(O)-R₅ and R₄ is -C(O)-R₆.

In one embodiment of the present invention, it is provided a composition (or a compound, use or method) described in the present specification, wherein R₅ is hydrogen.

In one embodiment of the present invention, it is provided a composition (or a compound, use or method) described in the present specification, wherein R₆ is hydrogen.

In a preferred embodiment of the present invention, it is provided a composition (or a compound, use or method) described in the present specification, wherein R₅ is hydrogen and R₆ is hydrogen.

In one embodiment of the present invention, it is provided a composition described in the present specification, which contains a compound having a structure of the formula II, or a stereoisomer thereof, or a salt thereof.

In one embodiment of the present invention, it is provided a composition (or a compound, use or method) described in the present specification, wherein A is the following:

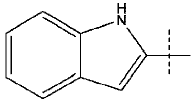

In one embodiment of the present invention, it is provided a composition (or a compound, use or method) described in the present specification, wherein B is the following: 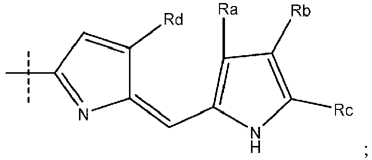 or

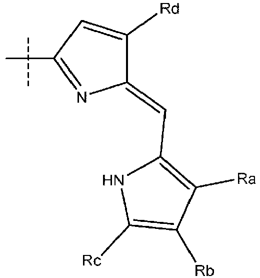

In a preferred embodiment of the present invention, it is provided a composition (or a compound, use or method) described in the present specification, wherein A is the following: 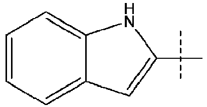 and B is the following: 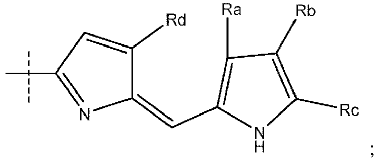 or

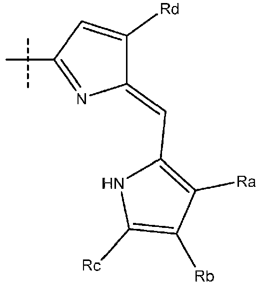

In a preferred embodiment of the present invention, it is provided a composition (or a compound, use or method) described in the present specification, wherein A-B is the following: 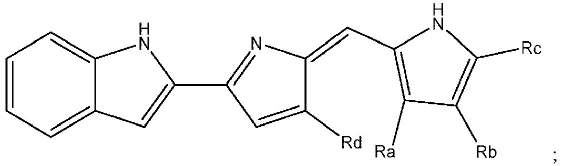 or

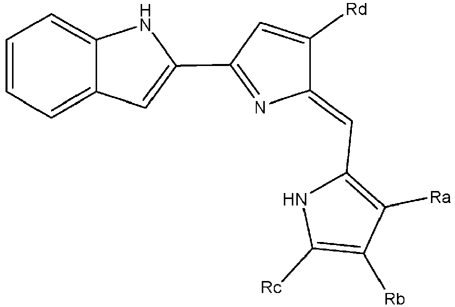

In one embodiment of the present invention, it is provided a composition (or a compound, use or method) described in the present specification, wherein A is the following:

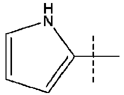

In one embodiment of the present invention, it is provided a composition (or a compound, use or method) described in the present specification, wherein B is the following: 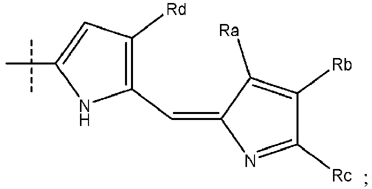 or

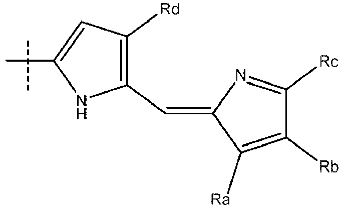

In a preferred embodiment of the present invention, it is provided a composition (or a compound, use or method) described in the present specification, wherein A is the following: 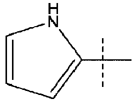 and B is the following: 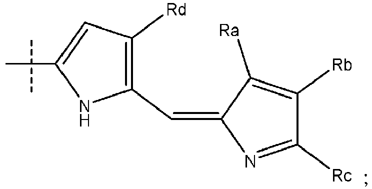 or

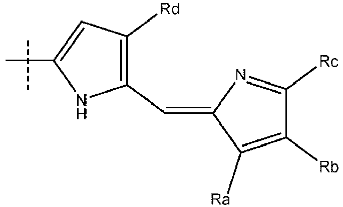

In a preferred embodiment of the present invention, it is provided a composition (or a compound, use or method) described in the present specification, wherein A-B is the following: 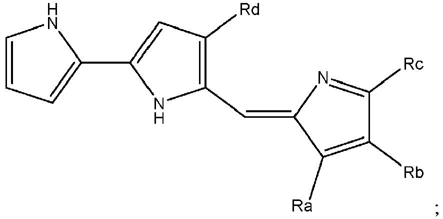 or

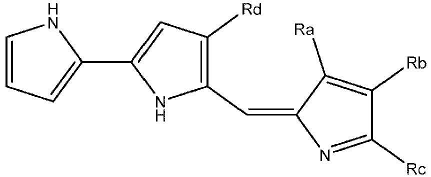

In a preferred embodiment of the present invention, it is provided a composition (or a compound, use or method) described in the present specification, wherein A is the following: 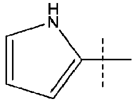 and B is the following: 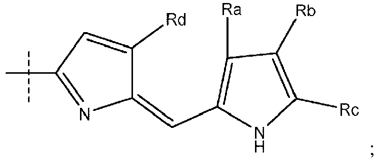 or

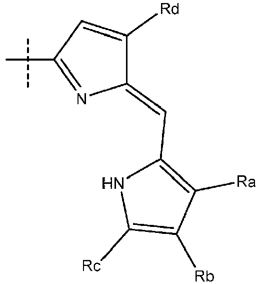

In a preferred embodiment of the present invention, it is provided a composition (or a compound, use or method) described in the present specification, wherein A-B is the following: 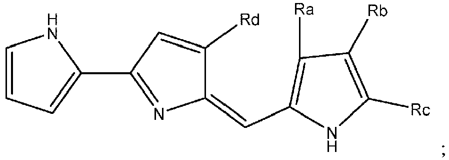 or

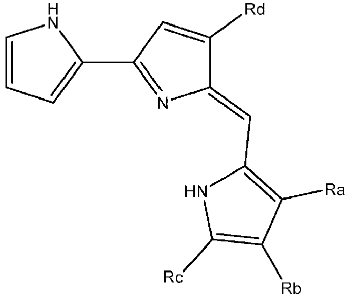

In one embodiment of the present invention, it is provided a composition (or a compound, use or method) described in the present specification, wherein Rd is C₁-C₆ alkoxy.

In a preferred embodiment of the present invention, it is provided a composition (or a compound, use or method) described in the present specification, wherein Rd is methoxy.

In one embodiment of the present invention, it is provided a composition (or a compound, use or method) described in the present specification, wherein the compound having a structure of the formula I or the compound having a structure of the formula II is selected from the group consisting of the following: 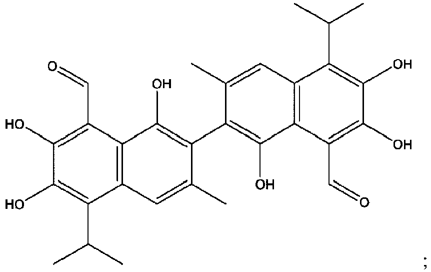 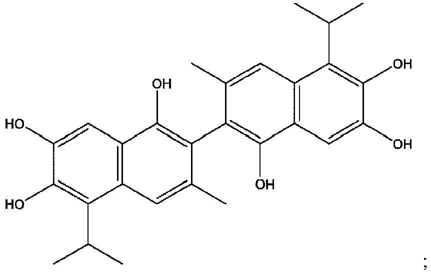 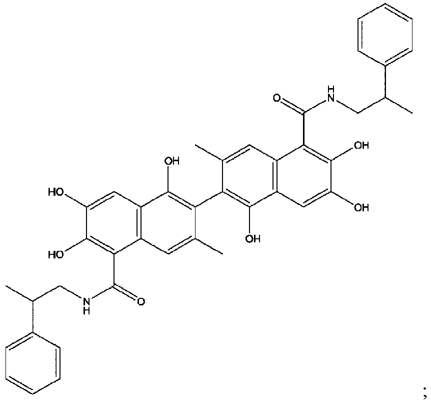 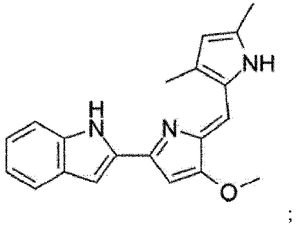 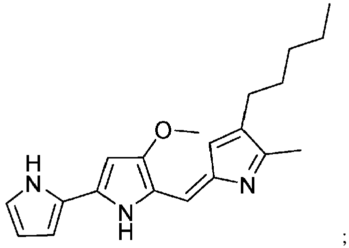 and

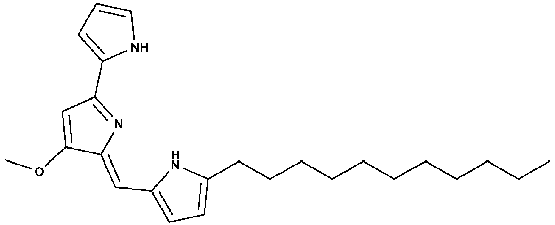

In a preferred embodiment of the present invention, it is provided a composition (or a compound, use or method) described in the present specification, wherein the compound having a structure of the formula I or the compound having a structure of the formula II is selected from the group consisting of (±)-gossypol, (S)-gossypol, (+)-apogossypol, (R)-(-)-gossypol, sabutoclax, obatoclax, prodigiosin and undecylprodigiosin.

In one embodiment of the present invention, it is provided a composition (or a compound, use or method) described in the present specification, which contains cucurbitacin, or a stereoisomer thereof, or a salt thereof.

In one embodiment of the present invention, it is provided a composition (or a compound, use or method) described in the present specification, wherein cucurbitacin is selected from the group consisting of cucurbitacin A, cucurbitacin B, cucurbitacin C, cucurbitacin D, cucurbitacin E, cucurbitacin F, cucurbitacin G, cucurbitacin H, cucurbitacin I, cucurbitacin J, cucurbitacin K, cucurbitacin L, cucurbitacin M, cucurbitacin N, cucurbitacin O, cucurbitacin P, cucurbitacin Q, cucurbitacin R, cucurbitacin S and cucurbitacin T.

In a preferred embodiment of the present invention, it is provided a composition (or a compound, use or method) described in the present specification, wherein the cucurbitacin is cucurbitacin B.

The compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof are commercially available, or can be produced by a well-known or universally known method or a method similar thereto. For example, these compounds can be purchased from MedChem Express.

The terms "extracellular vesicles" as used in the present specification are not particularly limited as long as they are vesicles secreted from cells, and there may be mentioned, for example, Exosomes, Microvesicles (MV), Apoptotic Bodies, etc.

The term "exosomes" as used in the present specification means vesicles of about 20 to about 200 nm derived from endocytosis pathways. As the constitutional components of exosomes, there may be mentioned, for example, proteins, nucleic acids (mRNA, miRNA, non-coding RNA), etc. Exosomes can have a function of controlling intercellular communication. Examples of the marker molecule of exosomes may be mentioned, for example, Alix, Tsg101, tetraspanin (for example, CD81, CD63, CD9), flotillin, phosphatidylserine, etc.

The term "microvesicles" as used in the present specification means vesicles of about 50 to about 1,000 nm derived from cytoplasmic membrane. As the constitutional components of the microvesicles, there may be mentioned, for example, proteins, nucleic acids (mRNA, miRNA, non-coding RNA, etc.), etc. Microvesicles can have a function of controlling intercellular communication, etc. Examples of the marker molecule of microvesicles may be mentioned, for example, integrin, selectin, CD40, CD154, etc.

The terms "apoptotic body" as used in the present specification mean vesicles of about 500 to about 2,000 nm derived from cytoplasmic membrane. As the constitutional components of the apoptotic body, there may be mentioned, for example, fragmented nuclei, cell organ (organelles), etc. Apoptotic body can have a function of inducing phagocytosis, etc. Examples of the marker molecule of apoptotic body may be mentioned, for example, Annexin V, phosphatidylserine, etc.

In one embodiment of the present invention, it is provided a composition (or a compound, use or method) described in the present specification, wherein the extracellular vesicles are exosomes.

The cells that secrete extracellular vesicles are not particularly limited as long as they can secrete extracellular vesicles, and there may be mentioned, for example, animal-derived cells including skin cells such as epidermal cells (keratinocytes, etc.), pigment cells (melanocytes, etc.), basal cells, prickle cells, granule cells, corneocytes, fibroblasts, mast cells, etc.; brain cells such as neural stem cells, neuroglial cells, nerve cells, microglia, etc.; cells derived from adipose tissues such as adipose cells (including white adipose cells, brown adipose cells, etc.), mesenchymal stem cells, etc.; lymphocytes, epithelial cells, endothelial cells, muscle cells, nerve cells, fibroblasts, hair cells, hepatocytes, gastric mucosal cells, intestinal cells, splenic cells, pancreatic cells (pancreatic exocrine cells, etc.), pneumocytes, nephrocytes, mesenchymal cells, etc., other than the above; tissue precursor cells; hematopoietic stem cells, mesenchymal stem cell (including those derived from bone marrow, those derived from placental tissue, those derived from umbilical cord tissue, those derived from dental pulp, those derived from synovial membrane, etc.), other tissue stem cells (somatic stem cells); etc., and plant-derived cells such as soft tissue cells, collenchyma tissue cells, sclerenchyma tissue cells, xylem cells, phloem cells, epidermal cells, etc., and these may be cells in organisms, primary cultured cells, subcultured cells or established cells, and these may be normal cells, diseased cells including cancerous or tumorigenic cells. The cells that secrete extracellular vesicles are preferably animal-derived cells, more preferably brain cells, cells derived from adipose tissues or mesenchymal stem cells, further preferably cells derived from adipose tissues or mesenchymal stem cells, and still further preferably mesenchymal stem cells.

In one embodiment of the present invention, it is a composition (or a compound, use or method) described in the present specification for promoting secretion of extracellular vesicles from animal-derived cells.

In one embodiment of the present invention, it is a composition (or a compound, use or method) described in the present specification for promoting secretion of extracellular vesicles from brain cells.

In one embodiment of the present invention, it is a composition (or a compound, use or method) described in the present specification for promoting secretion of extracellular vesicles from cells derived from adipose tissues.

In one embodiment of the present invention, it is a composition (or a compound, use or method) described in the present specification for promoting secretion of extracellular vesicles from mesenchymal stem cells.

The composition (or a compound) of the present invention can promote secretion of extracellular vesicles, so that it can be used for the treatment and/or prophylaxis of diseases in which extracellular vesicles may be involved.

Diseases that may be involved in extracellular vesicles include, which are not limited to these, for example, nervous diseases (including neurodegenerative diseases, nervous disorders, etc.), cancer, heart diseases, immune system diseases, kidney diseases, fibrotic diseases, metabolic diseases, eye diseases, etc. In addition, it includes other diseases in which extracellular vesicles may be involved such as wounds, osteoarthritis, bone lesion, musculoskeletal defects, alopecia, etc.

In one embodiment of the present invention, it is provided a composition (or a compound, use or method) described in the present specification for the treatment and/or prophylaxis of nervous diseases (including neurodegenerative diseases, nervous disorders, etc.), cancer, heart diseases, immune system diseases, kidney diseases, fibrotic diseases, metabolic diseases, eye diseases, wounds, osteoarthritis, bone lesion, musculoskeletal defects or alopecia.

In another embodiment of the present invention, it is provided a use of the compound described in the present specification for the manufacture of a medicament for the treatment and/or prophylaxis of nervous diseases (including neurodegenerative diseases, nervous disorders, etc.), cancer, heart diseases, immune system diseases, kidney diseases, fibrotic diseases, metabolic diseases, eye diseases, wounds, osteoarthritis, bone lesion, musculoskeletal defects or alopecia.

In another embodiment of the present invention, it is provided a method for the treatment and/or prophylaxis of nervous diseases (including neurodegenerative diseases, nervous disorders, etc.), cancer, heart diseases, immune system diseases, kidney diseases, fibrotic diseases, metabolic diseases, eye diseases, wounds, osteoarthritis, bone lesion, musculoskeletal defects or alopecia, which comprises administering the compound described in the present specification to a subject who requires it.

As the neurodegenerative diseases, which are not limited to these, there may be mentioned, for example, Alzheimer's disease, Parkinson's disease, cerebral atrophic lateral sclerosis, spinocerebellar degeneration, frontotemporal lobar degeneration, progressive supranuclear palsy, corticobasal degeneration, Huntington's disease, dystonia, prion disease, multiple-system atrophy, Lewy body disease, polyglutamine disease, etc.

As the nervous disorders, which are not limited to these, there may be mentioned, for example, traumatic brain injury, spinal cord injury, injury due to cerebral infarction, etc.

As the cancers, any of the solid cancers and blood cancers are contained, which are not limited to these, and there may be mentioned, for example, small cell lung cancer, non-small cell lung cancer, breast cancer, esophageal cancer, stomach cancer, small intestine cancer, large intestine cancer, colon cancer, rectal cancer, pancreatic cancer, prostatic cancer, bone marrow cancer, kidney cancer (including nephrocyte cancer, etc.), parathyroid cancer, adrenal cancer, ureter cancer, liver cancer, bile duct cancer, uterine cervix cancer, ovarian cancer (for example, its tissue type is serous adenocarcinoma, mucous adenocarcinoma, clear cell adenocarcinoma, etc.), testicular cancer, bladder cancer, vulvar cancer, penile cancer, thyroid cancer, head and neck cancer, craniopharyngeal cancer, pharyngeal cancer, tongue cancer, skin cancer, Merkel cell cancer, melanoma (malignant melanoma, etc.), epithelial cancer, squamous epithelial cell cancer, basal cell cancer, childhood cancer, unknown primary cancer, fibrosarcoma, mucosal sarcoma, liposarcoma, chondrosarcoma, osteogenic sarcoma, chordoma, angiosarcoma, lymphangiosarcoma, lymphangioendothelioma, Kaposi's sarcoma, leiomyosarcoma, rhabdomyosarcoma, synovioma, mesothelioma, Ewing sarcoma, seminoma, Wilms tumor, brain tumor, glioma, glioblastoma, astrocytoma, myeloblastoma, meningioma, neuroblastoma, medulloblastoma, retinoblastoma, spinal neoplasm, malignant lymphoma (for example, non-Hodgkin lymphoma, Hodgkin lymphoma, etc.), monocytic leukemia (chronic or acute), chronic or acute lymphocytic leukemia, adult T cell leukemia, etc.

As the heart diseases, which are not limited to these, there may be mentioned, for example, myocardial infarction, ischemic heart diseases, congested heart failure, arrhythmia, hypertrophic cardiomyopathy, dilated cardiomyopathy, myocarditis, valvular disease, etc.

As the immune system diseases, which are not limited to these, there may be mentioned, for example, graft-versus-host disease (see Giebel B et al. Stem Cell Investig. 2017 Oct 24; 4: 84, etc.), inflammatory bowel disease, rheumatoid arthritis, systemic lupus erythematosus, etc.

As the kidney diseases, which are not limited to these, there may be mentioned, for example, acute kidney injury, nephritis, chronic kidney disease, diabetic nephropathy, multiple cyst, etc.

As the fibrotic diseases, which are not limited to these, there may be mentioned, for example, hepatic fibrosis, hepatic cirrhosis, pulmonary fibrosis, idiopathic pulmonary fibrosis, kidney fibrosis, etc.

As the metabolic diseases, which are not limited to these, there may be mentioned, for example, obesity, diabetes, etc.

As the eye diseases, which are not limited to these, there may be mentioned, for example, visual impairment, myopia, age-related macular degeneration, etc.

The composition (or the compound) described in the present specification may be directly administered to a subject to promote secretion of extracellular vesicles to the subject (preferably for treating or preventing diseases in which the extracellular vesicles may be involved).

As the subject to be tested which became a subject to which secretion of extracellular vesicles is suppressed, which are not limited to these, there may be mentioned, for example, animals such as mammals, etc., including rodents such as mice, rats, hamsters, guinea pigs, etc.; Lagomorpha such as rabbits, etc.; ungulates such as pigs, cows, goats, horses, sheep, etc.; Carnivora such as dogs, cats, etc.; primates such as humans, monkeys, rhesus monkey, crab-eating monkey, marmoset, orangutan, chimpanzee; and plants, preferably animals, more preferably rodents or primates, further preferably primates, and more further preferably humans.

The composition (or the compound) described in the present specification may be contacted with cells, etc., *in vitro* or *ex vivo,* etc. Or else, the cells may be cultured in the composition (or the compound) described in the present specification *in vitro* or *ex vivo,* etc.

Accordingly, in one embodiment of the present invention, the composition described in the present specification is a medium composition.

For example, when the composition described in the present specification is used as a medium composition, in addition to the compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof, as long as the object of the present invention can be achieved, depending on necessity, for example, there may be added basal medium, sera, growth factors, iron sources, polyamines, minute amount metals, sugars, organic acids, amino acids and their derivatives, reducing agents, vitamins and their derivatives, steroids, antibiotics, buffers, inorganic salts, pH regulators, proteins (including enzymes, etc.), additives such as various kinds of activators and inhibitors, etc. Amounts of the basal medium and the additives are not particularly limited as long as the object of the present invention can be achieved, and can be appropriately selected by those skilled in the art.

As the basal medium, which is not limited to these, there may be mentioned, for example, DMEM, EMEM, IMDM (Iscove's Modified Dulbecco's Medium), GMEM (Glasgow's MEM), RPMI-1640, α-MEM, Ham's medium F-10, Ham's medium F-12, Ham's medium F12K, Medium 199, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's MB752/1, CMRL-1066, Williams' medium E, Brinster's BMOC-3 medium, E8 medium (Nature Methods, 2011, 8, 424-429), ReproFF, ReproFF2, etc., and these may be used alone or may be used in combination of two or more kinds.

As the sera, which are not limited to these, there may be mentioned, for example, sera derived from mammals such as fetal bovine serum, calf serum, horse serum, human serum, etc., and these may be used alone or may be used in combination of two or more kinds.

As the growth factors, which are not limited to these, there may be mentioned, for example, insulin, insulin-like growth factor (IGF), epithelial growth factor (EGF), nerve growth factor (NGF), brain-derived neurotrophic factor (BDNF), vascular endothelial cell growth factor (VEGF), granulocyte colony stimulating factor (G-CSF), granulocyte-macrophage colony-stimulating factor (GM-CSF), erythropoietin (EPO), thrombopoietin (TPO), hepatocyte growth factor (HGF), platelet-derived growth factor (PDGF), transforming growth factor beta (TGF-β), basic fibroblast growth factor (bFGF), etc., and these may be used alone or may be used in combination of two or more kinds.

As the iron sources, which are not limited to these, there may be mentioned, for example, transferrin, ferritin, iron (II) sulfate, etc., and these may be used alone or may be used in combination of two or more kinds.

As the polyamines, which are not limited to these, there may be mentioned, for example, spermine, spermidine, norspermine, norspermidine, homospermidine, homospermidine, cadaverine, putrescine, agmatine, ornithine, etc., and these may be used alone or may be used in combination of two or more kinds.

As the minute amount metals, which are not limited to these, there may be mentioned, for example, magnesium, zinc, cobalt, tin, molybdenum, nickel, selenium and its related substances (including sodium selenite, etc.), etc., and these may be used alone or may be used in combination of two or more kinds.

As the sugars, which are not limited to these, there may be mentioned, for example, glucose, galactose, fructose, sucrose, etc., and these may be used alone or may be used in combination of two or more kinds.

As the organic acids, which are not limited to these, there may be mentioned, for example, pyruvic acid, lactic acid, linoleic acid, linolic acid, etc., and these may be used alone or may be used in combination of two or more kinds.

As the amino acids and their derivatives, which are not limited to these, there may be mentioned, for example, glycine, L-alanine, L-serine, L-valine, L-leucine, L-isoleucine, L-arginine, L-lysine, L-asparagine, L-glutamine, L-aspartic acid, L-glutamic acid, L-methionine, L-cysteine, L-proline, L-threonine, L-histidine, L-tryptophan, L-phenylalanine, L-tyrosine, L-carnitine, L-ornithine, glutathione (including the reduced form), etc., and these may be used alone or may be used in combination of two or more kinds.

As the reducing agents, which are not limited to these, there may be mentioned, for example, 2-mercaptoethanol, dithiothreitol, etc., and these may be used alone or may be used in combination of two or more kinds.

As the vitamins and their derivatives, which are not limited to these, there may be mentioned, for example, vitamin A and its derivatives (including vitamin A acetate ester, etc.), vitamin B1, vitamin B2, vitamin B3, vitamin B5, vitamin B6, vitamin B12, vitamin C, vitamin D, vitamin E and its derivatives (including DL-α-tocopherol acetate, etc.), vitamin K, biotin, folic acid, etc., and these may be used alone or may be used in combination of two or more kinds.

As the steroids, which are not limited to these, there may be mentioned, for example, β-estradiol, progesterone, corticosterone, etc., and these may be used alone or may be used in combination of two or more kinds.

As the antibiotics, which are not limited to these, there may be mentioned, for example, penicillin, streptomycin, gentamicin, kanamycin, etc., and these may be used alone or may be used in combination of two or more kinds.

As the buffers, which are not limited to these, there may be mentioned, for example, sodium hydrogen carbonate, disodium hydrogen phosphate, sodium dihydrogen phosphate, HEPES, etc., and these may be used alone or may be used in combination of two or more kinds.

As the inorganic salts, which are not limited to these, there may be mentioned, for example, sodium chloride, calcium chloride, potassium chloride, copper sulfate, iron (II) nitrate, iron sulfate, magnesium chloride, magnesium sulfate, sodium hydrogen carbonate, disodium hydrogen phosphate, sodium dihydrogen phosphate, etc., and these may be used alone or may be used in combination of two or more kinds.

As the pH regulators, which are not limited to these, there may be mentioned, for example, hydrochloric acid, nitric acid, phosphoric acid, disodium hydrogen phosphate, sodium dihydrogen phosphate, sodium hydroxide, potassium hydroxide, sodium hydrogen carbonate, etc., and these may be used alone or may be used in combination of two or more kinds.

As the proteins (including enzymes, etc.), which are not limited to these, there may be mentioned, for example, human albumin, bovine serum albumin, superoxide dismutase, catalase, etc., and these may be used alone or may be used in combination of two or more kinds.

As the various kinds of activators and inhibitors, which are not limited to these, there may be mentioned, for example, Wnt signal activators such as Wnt-3a, Wnt-5a, lithium chloride, complement molecule C1q, etc.; Rho-kinase (ROCK) inhibitors such as Y-27632, K-115 (ripasudil hydrochloride hydrate), HA1077 (fasudil hydrochloride), etc.; and the like, and these may be used alone or may be used in combination of two or more kinds.

In another embodiment of the present invention, it is provided a method for promoting secretion of extracellular vesicles from cells *in vitro* or *ex vivo* which uses the composition (preferably a medium composition) (or a compound) described in the present specification.

In another preferred embodiment of the present invention, it is provided a method for promoting secretion of extracellular vesicles from cells *in vitro* or *ex vivo,* which comprises contacting the cells with the composition (preferably a medium composition) (or a compound) described in the present specification.

By using the methods described in the present specification, for example, extracellular vesicles can be secreted from cells in the culture supernatant.

Accordingly, in another embodiment of the present invention, it is provided a culture supernatant which can be obtained by the method described in the present specification.

In the culture supernatant described in the present specification, extracellular vesicles can be contained.

Accordingly, in another embodiment of the present invention, it is provided extracellular vesicles which can be obtained from the culture supernatant described in the present specification.

As stated above, when cells are cultured with the composition (or the compound) described in the present specification *in vitro* or *ex vivo,* for example, extracellular vesicles can be secreted in the culture supernatant. Accordingly, in another embodiment of the present invention, it is provided a method for producing extracellular vesicles *in vitro* or *ex vivo* which uses the composition (or the compound) described in the present specification. In addition, in another preferred embodiment of the present invention, it is provided a method for producing extracellular vesicles, which comprises bringing the composition (or the compound) described in the present specification into contact with cells (preferably culturing cells with the composition (or the compound) described in the present specification).

For example, when extracellular vesicles are contained in the culture supernatant, etc., the extracellular vesicles can be subjected to purification, concentration, isolation, etc., by a well-known or universally known method (for example, centrifugation, ultrafiltration, purification using an antibody, purification using a column, etc.).

The extracellular vesicles (or culture supernatants containing these, etc.) obtained by the method described in the present specification can be useful for the treatment and/or prophylaxis of other diseases in which the extracellular vesicles may be involved such as wounds, osteoarthritis, bone lesion, musculoskeletal defects, alopecia, etc., in addition to nervous diseases (including neurodegenerative diseases, nervous disorders, etc.), cancer, heart diseases, immune system diseases, kidney diseases, fibrotic diseases, metabolic diseases, eye diseases, etc.

In one embodiment of the present invention, it is provided a composition (for example, a pharmaceutical composition) containing the extracellular vesicles (or culture supernatants containing these, etc.) obtained by the method described in the present specification for the treatment and/or prophylaxis of nervous diseases (including neurodegenerative diseases, nervous disorders, etc.), cancer, heart diseases, immune system diseases, kidney diseases, fibrotic diseases, metabolic diseases, eye diseases, wounds, osteoarthritis, bone lesion, musculoskeletal defects or alopecia.

The composition described in the present specification may be used as a pharmaceutical or cosmetic composition. In addition, the culture supernatant described in the present specification may be used for a pharmaceutical or cosmetic purpose. Or else, for example, a material in which the extracellular vesicles are purified, concentrated, isolated, etc., from the culture supernatant described in the present specification by a well-known method, or a material in which the above is formulated, etc., may be used for a pharmaceutical or cosmetic use.

When the composition described in the present specification is used, for example, as a pharmaceutical or cosmetic composition, etc., in the composition described in the present specification, which are not limited to these, it may contain, for example, additives such as excipients, lubricants, binders, disintegrants, pH regulators, solvents, solubilizing aids, suspending agents, tonicity agents, buffers, analgesics, preservatives, antioxidants, colorants, sweeteners, surfactants, etc.

In addition, when a material in which the culture supernatant described in the present specification or a material in which the extracellular vesicles are purified, concentrated, isolated, etc., therefrom is used as, for example, a pharmaceutical or cosmetic composition, in the culture supernatant or a material in which the extracellular vesicles are purified, concentrated, isolated, etc., therefrom, which are not limited to these, it may contain, for example, additives such as excipients, lubricants, binders, disintegrants, pH regulators, solvents, solubilizing aids, suspending agents, tonicity agents, buffers, analgesics, preservatives, antioxidants, colorants, sweeteners, surfactants, etc.

As the excipients, which are not limited to these, there may be mentioned, for example, lactose hydrate, white sugar, glucose, starch, sucrose, crystalline cellulose, mannitol, etc., and these may be used alone or may be used in combination of two or more kinds.

As the lubricants, which are not limited to these, there may be mentioned, for example, light anhydrous silicic acid, stearic acid, magnesium stearate, calcium stearate, sucrose fatty acid ester, polyethylene glycol, talc, etc., and these may be used alone or may be used in combination of two or more kinds.

As the binders, which are not limited to these, there may be mentioned, for example, gum arabic, crystalline cellulose, white sugar, mannitol, dextrin, hydroxypropyl cellulose, hydroxymethyl cellulose, polyvinylpyrrolidone, etc., and these may be used alone or may be used in combination of two or more kinds.

As the disintegrants, which are not limited to these, there may be mentioned, for example, starch, carboxymethyl cellulose, carboxymethyl cellulose calcium, croscarmellose sodium, croscarmellose calcium, carboxymethyl starch sodium, crospovidone, low-substitution degree hydroxypropyl cellulose, etc., and these may be used alone or may be used in combination of two or more kinds.

As the pH regulators, which are not limited to these, there may be mentioned, for example, acetic acid, lactic acid, tartaric acid, oxalic acid, glycolic acid, malic acid, citric acid, succinic acid, fumaric acid, phosphoric acid, hydrochloric acid, sulfuric acid, nitric acid and a salt thereof, sodium hydroxide, potassium hydroxide, sodium hydrogen carbonate, potassium carbonate, etc., and these may be used alone or may be used in combination of two or more kinds.

As the solvents, which are not limited to these, there may be mentioned, for example, water such as tap water, normal water, distilled water, purified water, water for injection, etc.; alcohols such as methanol, ethanol, propanol, isopropanol, etc.; acetone; single fatty acids such as acetic acid, propanoic acid, butanoic acid, pentanoic acid, hexanoic acid, heptanoic acid, myristic acid, stearic acid, oleic acid, etc., or an ester thereof; vegetable oils such as sesame oil, peanut oil, coconut oil, palm oil, soybean oil, olive oil, coconut butter, corn oil, cottonseed oil, castor oil, rapeseed oil, sunflower oil, etc.; propylene glycol; macrogol, etc., and these may be used alone or may be used in combination of two or more kinds.

As the solubilizing aids, which are not limited to these, there may be mentioned, for example, polyethylene glycol; propylene glycol; cyclodextrin; sugar alcohols such as mannitol, etc.; benzyl benzoate; trisaminomethane; cholesterol; triethanolamine; sodium carbonate; sodium citrate; alcohols such as methanol, ethanol, propanol, isopropanol, etc.; single fatty acids such as acetic acid, propanoic acid, butanoic acid, pentanoic acid, hexanoic acid, heptanoic acid, myristic acid, stearic acid, oleic acid, etc., or an ester thereof; vegetable oils such as sesame oil, peanut oil, coconut oil, palm oil, soybean oil, olive oil, coconut butter, corn oil, cottonseed oil, castor oil, rapeseed oil, sunflower oil, etc., and these may be used alone or may be used in combination of two or more kinds.

As the suspending agents, which are not limited to these, there may be mentioned, for example, stearyltriethanolamine, sodium lauryl sulfate, laurylamino-propionic acid, lecithin, glycerin monostearate, polyvinyl alcohol, polyvinylpyrrolidone, carboxymethyl cellulose sodium, methyl cellulose, hydroxymethyl cellulose, hydroxyethyl cellulose, hydroxypropyl cellulose, etc., and these may be used alone or may be used in combination of two or more kinds.

As the tonicity agents, which are not limited to these, there may be mentioned, for example, sodium chloride, glycerin, mannitol, etc., and these may be used alone or may be used in combination of two or more kinds.

As the buffers, which are not limited to these, there may be mentioned, for example, buffer solutions such as phosphates, acetates, carbonates, citrates, etc., and buffer solutions containing these, etc., and these may be used alone or may be used in combination of two or more kinds.

As the analgesics, which are not limited to these, there may be mentioned, for example, benzyl alcohol, etc.

As the preservatives, which are not limited to these, there may be mentioned, for example, sorbic acid, potassium sorbate, calcium sorbate, benzoic acid, sodium benzoate, propionic acid, sodium propionate, calcium propionate, sodium dehydroacetate, natamycin, pimaricin, polylysine, nisin, isopropyl paraoxybenzoate, isopropyl parahydroxybenzoate, isopropylparaben, etc., and these may be used alone or may be used in combination of two or more kinds.

As the antioxidants, which are not limited to these, there may be mentioned, for example, sulfites, ascorbic acid, etc., and these may be used alone or may be used in combination of two or more kinds.

As the colorants, which are not limited to these, there may be mentioned, for example, yellow ferric oxide, black iron oxide, food yellow No.4, food red No.3, tar pigment, caramel, titanium oxide, riboflavins, etc., and these may be used alone or may be used in combination of two or more kinds.

As the sweeteners, which are not limited to these, there may be mentioned, for example, sugars such as sucrose, fructose, etc.; sugar alcohols such as xylitol, sorbitol, etc., and these may be used alone or may be used in combination of two or more kinds.

As the surfactants, which are not limited to these, there may be mentioned, for example, polysorbates, sodium lauryl sulfate, polyoxyethylene hydrogenated castor oil, etc., artificial sweeteners such as aspartame, acesulfame potassium, sucralose, etc., and these may be used alone or may be used in combination of two or more kinds.
the composition described in the present specification, or the culture supernatant described in the present specification or a material in which the extracellular vesicles are purified, concentrated, isolated, etc., therefrom can be formulated with the above-mentioned additives by the methods known *per se*, for example, to formulations such as tablets, coated tablets, orally disintegrating tablets, chewable agents, pills, granules, fine granules, powders, hard capsules, soft capsules, liquids (for example, including syrups, injections, lotions, etc.), suspensions, emulsions, jelly agents, patches, ointments, creams, inhalants, suppositories, etc. These may be oral preparations or parenteral preparations. The formulated material may contain, not only the compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof, or the culture supernatant described in the present specification or a material in which the extracellular vesicles are purified, concentrated, isolated, etc., therefrom, but also beneficial other components (for example, pharmaceutically or cosmetically beneficial other components), depending on the purpose.

In the case of a tablet, for example, it can be formulated as follows.

The compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof, the culture supernatant described in the present specification or a material in which the extracellular vesicles are purified, concentrated, isolated, etc., therefrom, and an excipient, a disintegrator, a binder, etc., are mixed and granulated with water. The obtained granules are dried and the granules are pulverized depending on necessity. Then, a lubricant, etc., is added thereto and the mixture is further mixed, and this is subjected to compression molding to obtain a tablet.

In the case of a hard capsule, for example, it can be formulated as follows.

The compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof, the culture supernatant described in the present specification or a material in which the extracellular vesicles are purified, concentrated, isolated, etc., therefrom, and an excipient, etc., are mixed, and a lubricant, etc., is added thereto and the mixture is further mixed. Then, the obtained mixture is filled in a hard capsule (for example, a gelatin capsule, etc.) to obtain a hard capsule.

In the case of an injection, for example, it can be formulated as follows.

The compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof, the culture supernatant described in the present specification or a material in which the extracellular vesicles are purified, concentrated, isolated, etc., therefrom, and a solubilizing aid, water, etc., are mixed, and after adding thereto a pH regulator, etc., water is further added to adjust it to a desired volume. By subjecting the material to filtration, sterilization, etc., depending on necessity, an injection can be obtained.

A content of the compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof in the composition described in the present specification or in the formulation thereof can be, for example, about 0.01 to about 99.9% by mass, preferably about 0.1 to about 80% by mass, and more preferably about 1% to about 50% by mass based on the entire composition or the formulation thereof. Alternatively, a content of the compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof in the composition described in the present specification or in the formulation thereof is, for example, about 0.001 to about 1,000 µmol, preferably about 0.01 to about 100 µmol, and more preferably about 0.1 to about 50 µmol based on the entire composition or the formulation thereof.

A content of extracellular vesicles in the culture supernatant described in the present specification or a material in which the extracellular vesicles are purified, concentrated, isolated, etc., therefrom, or the formulation thereof can be, for example, about 0.01 to about 99.9% by mass, preferably about 0.1 to about 80% by mass, and more preferably about 1 to about 50% by mass based on the entire culture supernatant described in the present specification or a material in which the extracellular vesicles are purified, concentrated, isolated, etc., therefrom, or the formulation thereof.

An administration dose of the composition described in the present specification or a formulation thereof can be appropriately determined in consideration of sex, age, body weight, health condition, degree of medical condition or diet of the subject to be administered; administration time; administration method; combination of the other drugs; and other factors.

An administration dose of the composition described in the present specification or a formulation thereof is not specifically limited and, for example, as the compound having a structure of the formula I, a racemate thereof or a stereoisomer thereof, or a salt thereof, or the compound having a structure of the formula II, or a stereoisomer thereof, or a salt thereof, or cucurbitacin, or a stereoisomer thereof, or a salt thereof, it can be about 0.01 to about 10 mg/kg body weight, preferably about 0.05 to about 5 mg/kg body weight, more preferably about 0.1 to about 1 mg/kg body weight, per a day. These may be administered in a single dose, or may be administered by dividing into two or more times. Provided that, depending on necessity, it may exceed the range of the above-mentioned administration dose.

The administration schedule can be determined in consideration of the sex, age, body weight, health condition, degree of medical condition or diet of the subject to be administered; administration time; administration method; combination with other medicines; and other factors, and may be mentioned, for example, every day, once every two days, once every three days, once a week, once a month, once every three months, once every six months, etc.

An administration dose of the culture supernatant described in the present specification or a material in which the extracellular vesicles are purified, concentrated, isolated, etc., therefrom, or the formulation thereof can be appropriately determined in consideration of sex, age, body weight, health condition, degree of medical condition or diet of the subject to be administered; administration time; administration method; combination of the other drugs; and other factors.

An administration dose of the culture supernatant described in the present specification or a material in which the extracellular vesicles are purified, concentrated, isolated, etc., therefrom, or the formulation thereof is not specifically limited. These may be administered in a single dose, or may be administered by dividing into two or more times.

The administration schedule can be determined in consideration of the sex, age, body weight, health condition, degree of medical condition or diet of the subject to be administered; administration time; administration method; combination with other medicines; and other factors, and may be mentioned, for example, every day, once every two days, once every three days, once a week, once a month, once every three months, once every six months, etc.

### EXAMPLES

Hereinafter, the present invention will be explained in more detail by referring to Examples, but these Examples do not limit the scope of the present invention at all.

### [Example 1] (±)-gossypol acetate

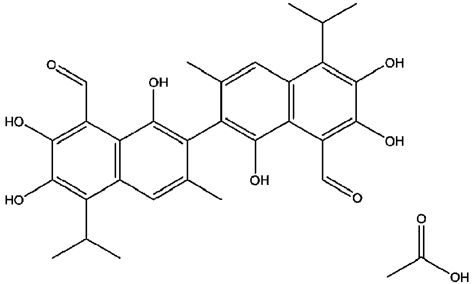

As the compound of Examples 1, a compound produced by MedChem Express (Product code: HY-17510, CAS Registry Number: 12542-36-8) was used.

### [Example 2] (S)-gossypol acetate

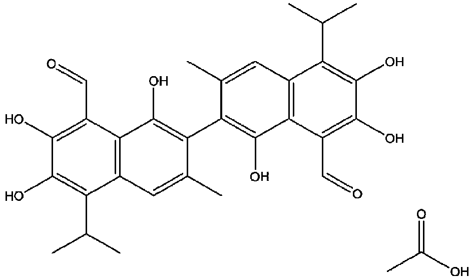

As the compound of Examples 2, a compound produced by MedChem Express (Product code: HY-1546D, CAS Registry Number: 1189561-66-7) was used.

### [Example 3] (+)-apogossypol

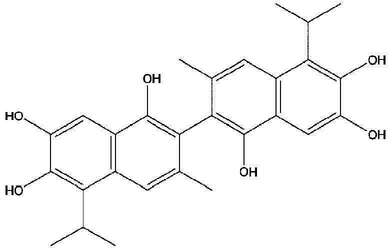

As the compound of Examples 3, a compound produced by Apex Bio (Product code: B4902, CAS Registry Number: 66389-74-0) was used.

### [Example 4] (R)-(-)-gossypol

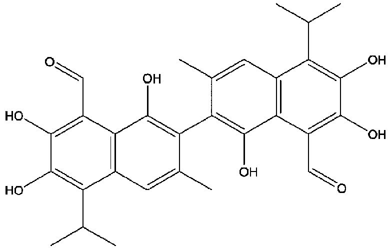

As the compound of Examples 4, a compound produced by Sigma (Product code: SML0433, CAS Registry Number: 90141-22-3) was used.

### [Example 8] Sabutoclax

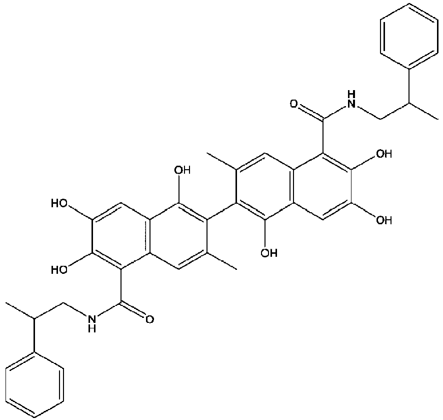

As the compound of Examples 8, a compound produced by Apex Bio (Product code: A4199, CAS Registry Number: 1228108-65-3) was used.

### [Example 5] Obatoclax mesylate

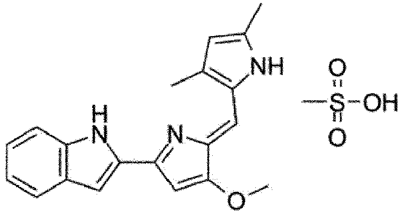

As the compound of Examples 5, a compound produced by Santa cruz (Product code: sc-364221, CAS Registry Number: 803712-79-0) was used.

### [Example 6] Prodigiosin hydrochloride

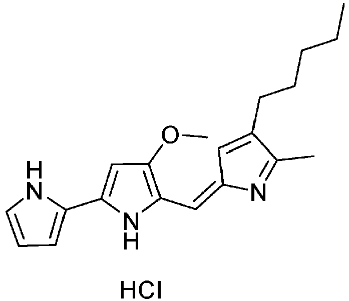

As the compound of Examples 6, a compound produced by Sigma (Product code: P0103, CAS Registry Number: 56144-17-3) was used.

### [Example 7] Undecylprodigiosin hydrochloride

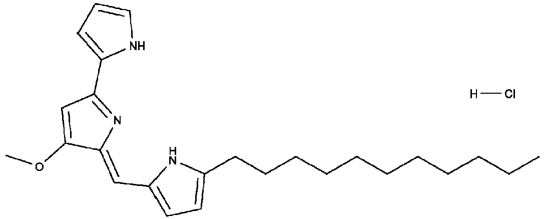

As the compound of Examples 7, a compound produced by Sigma (Product code: SML1576, CAS Registry Number: 56247-02-0) was used.

### [Example 9] Cucurbitacin B

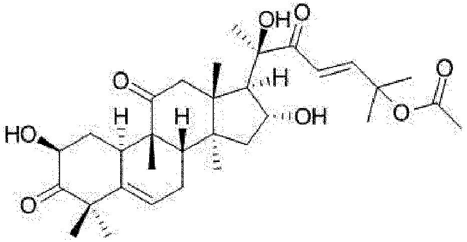

As the compound of Examples 9, a compound produced by MedChem Express (commodity code: HY-N0416, CAS Registry Number: 6199-67-3) was used.

### [Test Example 1: Evaluation 1 of promotion of secretion of extracellular vesicles]

### <Preparation of culture supernatant>

After stirring 10 mL of deactivated FBS and 2 mL of a 50% (w/v%) Poly(ethylene glycol) 10,000 solution (#81280 available from Sigma-Aldrich) at 4°C for 2 hours, extracellular vesicles were precipitated under the centrifugation conditions of 1,500×g at 4°C for 30 minutes, and the supernatant was recovered to prepare Exosome-free FBS. Next, U-87 MG cells (HTB-14TM available from ATCC) were suspended in Advanced DMEM (#12491015 available from Thermo Fisher Scientific K.K.) containing 0.03% (w/v%) SphereMax (trademark) (available from Nissan Chemical Corporation) (low molecular weight agar-containing medium composition described in Test Example 10 of WO 2016/167373) and 2% (v/v%) Exosome-free FBS with 2×10⁴ cells/81 µL, and seeded on an ultra-low adhesive surface 384-well black clear bottom plate (#3827 manufactured by Corning Inc.). To the seeded U-87 MG cells was added 9 µL (final concentration; Examples 1: 10 µM, Examples 5: 5 µM) of a compound solution of Examples 1 and 5 prepared in Advanced DMEM (containing 2% (v/v%) Exosome-free FBS), and the cells were cultured for 24 hours. Thereafter, the plate was centrifuged under the conditions of 1200xg at 4°C for 1 hour to precipitate the cells, and the culture supernatant was recovered. For the detection of extracellular vesicles contained in the culture supernatant, the Tim4-CD63 ELISA method or Tim4-CD9 ELISA method mentioned later was used.

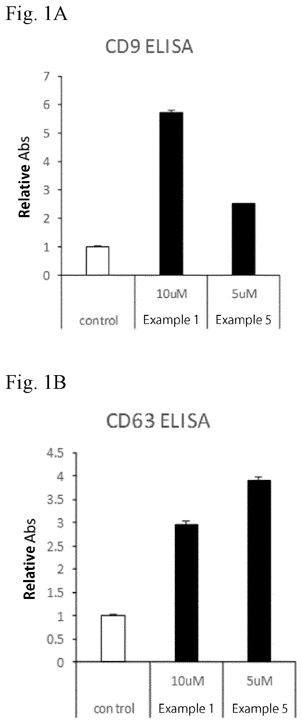

The results are shown in Fig. 1A and Fig. 1B.

### <Tim4-CD63 ELISA method>

To a 384-well plate (#464718 manufactured by Thermo Fisher Scientific K.K.) was added 1 µg/mL Tim4 protein prepared in Carbonate Buffer (a solution containing 71.4 mM NaHCO₃ and 28.6 mM Na₂CO₃), and it was made a solid phase. The 384-well plate was washed with a 0.05% (v/v%) Tween 20-containing TBS (Tris-Buffer Saline) solution (hereinafter sometimes referred to as "TBST solution".), and then, 50 µL/well of a TBST solution containing 1% (w/v%) BSA was added thereto to carry out a blocking treatment for 1 hour. Next, 30 µL of the culture supernatant recovered above was added to the well, and further 10 µL of an 8 mM CaCl₂ solution was added thereto to bind extracellular vesicles to the Tim4 protein. At this time, as a comparative object, a compound-untreated culture supernatant (solvent alone, control) was added to the Tim4 protein solid-phased plate in the same manner. Subsequently, 30 µL/well of 1 µg/mL Anti-human CD63 Antibody (#353014 available from BioLegend Inc.) diluted with a TBST solution containing 2 mM CaCl₂ was added thereto and the mixture was reacted for 1 hour. To the mixture was added 50 µL/well of a TBST solution containing 2 mM CaCl₂, and then, the TBST solution was removed. After repeating this operation three times, 30 µL/well of 80 ng/mL HRP-conjugated Anti-mouse IgG (#405306 available from BioLegend Inc.) diluted with a TBST solution containing 2 mM CaCl₂ was added thereto and the mixture was reacted for 1 hour. Finally, 30 µL/well of a TMB solution (#05298-80 available from Nacalai Tesque Inc.) was added to the mixture and the mixture was reacted for 30 minutes, and then, 30 µL/well of a 1M H₂SO₄ solution was added thereto to stop the reaction and by measuring an absorbance at 450 nm to detect the amount of the extracellular vesicles. A relative value when the compound-untreated group (control) was made 1 was calculated.

### <Tim4-CD9 ELISA method >

The Tim4-CD9 ELISA method was carried out in the same manner except that 1 µg/mL Anti-human CD63 Antibody in the above-mentioned Tim4-CD63 ELISA method was replaced with 0.5 µg/mL Anti-human CD9 Antibody (#312102 available from BioLegend Inc.).

### [Test Example 2: Evaluation 1 of cytotoxicity]

After stirring 10 mL of deactivated FBS and 2 mL of a 50 % (w/v%) Poly(ethylene glycol) 10,000 solution (#81280 available from Sigma-Aldrich Co., LLC) at 4°C for 2 hours, extracellular vesicles were precipitated under the centrifugation conditions of 1,500×g at 4°C for 30 minutes, and the supernatant was recovered to prepare Exosome-free FBS. U-87 MG cells (ATCC, HTB-14TM) were suspended in Advanced DMEM (#12491015 available from Thermo Fisher Scientific K.K.) containing 2% (v/v%) Exosome-free FBS in which 0.03% (w/v%) of SphereMax (trademark) (available from Nissan Chemical Corporation) (low molecular weight agar-containing medium composition described in Test Example 10 of WO 2016/167373) was contained therein with 2×10⁴ cells/81 µL, and seeded on an ultra-low adhesive surface 384-well black clear bottom plate (#3827 manufactured by Corning Inc.). To the seeded U-87 MG cells was added 9 µL (final concentration; Example 1:10 µM, Example 5: 5 µM) of the compound solution of Examples 1 and 5 prepared in Advanced DMEM (containing 2% (v/v%) Exosome-free FBS), and the cells were cultured for 24 hours. After 24 hours, the plate was subjected to centrifugation under the conditions of 1,200xg at 4°C for 1 hour to precipitate the cells, and 20 µL of the culture supernatant was transferred to a 384-well microplate (#781101 manufactured by Greiner bio-one). The activity of lactate dehydrogenase (LDH) contained in the culture supernatant was measured using Cytotoxicity LDH Assay Kit-WST (#CK12 manufactured by Dojindo Laboratories). 5 mL of Assay Buffer was added to 500 tests of Dye Mixture and dissolved to prepare Working Solution. To the 384-well microplate to which 20 µL of the culture supernatant was transferred was added 20 µL of the Working Solution, and after mixing well, a coloring reaction was carried out at room temperature for 30 minutes. The reaction was stopped by adding 10 µL of Stop Solution, and the cytotoxicity was evaluated by measuring the absorbance at 490 nm. The activities of LDH of the dead cell group (sample (Lysed cells) to which 9 µL of Lysis Buffer was added to compound-untreated cells 15 minutes before recovery of the culture supernatant) and the culture supernatant when the cells were cultured without adding any compound were also measured in the same manner, and the measured value of the positive control (Lysed cells) was evaluated as 100% cytotoxicity, and cytotoxicity of each compound was evaluated.

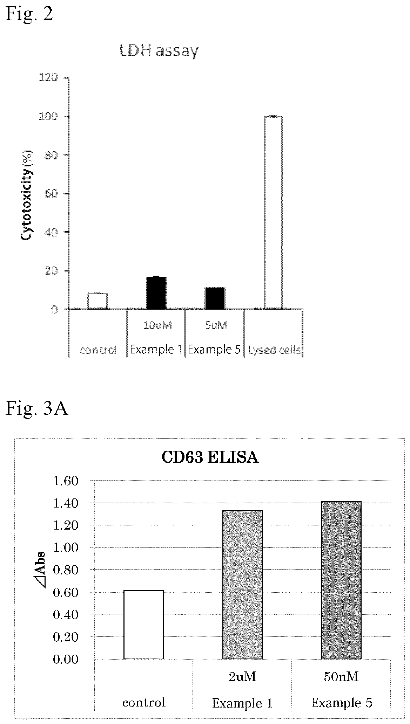

The results are shown in Fig. 2.

### [Test Example 3: Evaluation 2 of promotion of secretion of extracellular vesicles]

### <Preparation of culture solution>

Mesenchymal stem cells derived from the human adipose tissue (#C-12977 manufactured by Takara Bio Inc.) were suspended in mesenchymal stem cell growth medium 2 (C-28009 manufactured by Takara Bio Inc.) so as to be 8×10⁴ cells/1.8 mL, and seeded on a 6-well cell culture treatment plate (3516 manufactured by Corning Inc.). To the seeded mesenchymal stem cells derived from human adipose tissue was added 200 µL of the compound solution of each of Examples 1 to 8 diluted to 10-fold concentration with the mesenchymal stem cell growth medium 2, or mesenchymal stem cell growth medium alone (control), and after culturing for 24 hours, the culture solution was recovered. Subsequently, the recovered culture solution was centrifuged by 20,000xg at 4°C for 60 minutes to prepare a culture solution from which cell fragments and Large Extracellular Vesicles were removed. For the detection of the extracellular vesicles, PS Capture (trademark) exosome ELISA kit (anti-mouse IgG POD) (#297-79201 manufactured by FUJIFILM Wako Pure Chemical Corporation) was used.

### <Detection by PS Capture (trademark) exosome ELISA kit>

To Reaction/Washing Buffer (10×) diluted 10-fold with purified water was added 1/100 amount of Exosome Binding Enhancer (100×) to prepare a Reaction/Washing Buffer (1×). The culture solution prepared as mentioned above was diluted 5-fold with the Reaction/Washing Buffer (1×). After washing the Exosome Capture 96-well plate with 300 µL of the Reaction/Washing Buffer (1×) three times, 5-fold diluted each of the culture solution or mesenchymal stem cell growth medium alone in which no cell was cultured was added to each well, and the mixture was reacted at room temperature for 2 hours while shaking with a microplate shaker. After completion of the reaction, the reaction mixture was discarded, and after washing each well with 300 µL of the Reaction/Washing Buffer (1×) three times, 100 µL of Primary Antibody Anti-CD63 (×100) diluted 1,000-fold with the Reaction/Washing Buffer (1×) was added thereto, and the mixture was reacted at room temperature for 1 hour while shaking with a microplate shaker. After completion of the reaction, the reaction mixture was discarded, and after washing each well with 300 µL of the Reaction/Washing Buffer (1×) three times, 100 µL of the Secondary Antibody HRP-conjugated Anti-mouse IgG (×) diluted 1,000-fold with the Reaction/Washing Buffer (1×) was added thereto, and the mixture was reacted at room temperature for 1 hour while shaking with a microplate shaker. After completion of the reaction, the reaction mixture was discarded, and after washing each well with 300 µL of the Reaction/Washing Buffer (1×) five times, 100 µL of TMB Solution was added, and after shaking for 1 minute with a microplate shaker, the mixture was reacted at room temperature for 30 minutes. After completion of the reaction, 100 µL of Stop Solution was added thereto, and after shaking with a microplate shaker for 5 seconds, the absorbance at 450 nm and 620 nm was measured with Enspire (manufactured by PerkinElmer), and the absorbance at 620 nm was subtracted from the absorbance at 450 nm for each well.

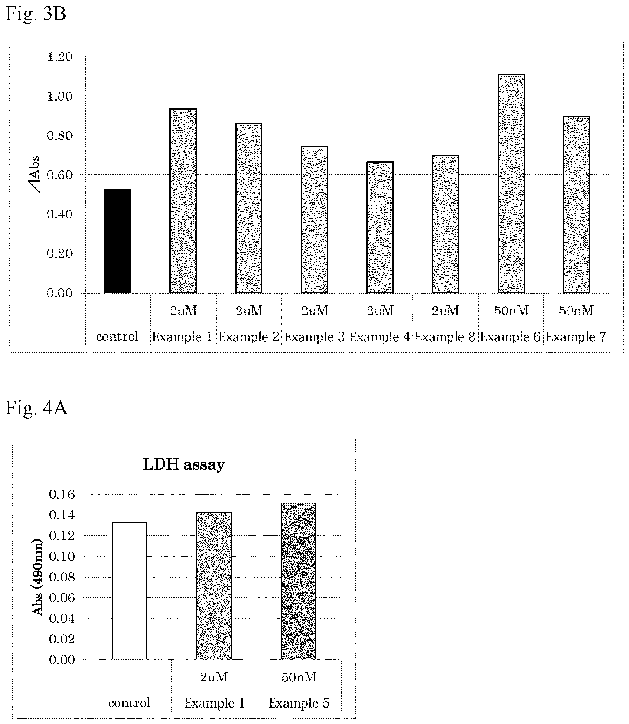

The results are shown in Table 1 and Table 2, and Fig. 3A and Fig. 3B.

**[Table 1]**

| | Concentration | Difference in absorbance |
|---|---|---|
| Example 1 | 2 µM | 2.15 |
| Example 5 | 50 nM | 2.27 |

**[Table 2]**

| | Concentration | Difference in absorbance |
|---|---|---|
| Example 1 | 2µM | 1.97 |
| Example 2 | 2 µM | 1.79 |
| Example 3 | 2 µM | 1.33 |
| Example 4 | 2 µM | 1.51 |
| Example 8 | 2 µM | 1.41 |
| Example 6 | 50 nM | 2.38 |
| Example 7 | 50 nM | 1.88 |

### [Test Example 4: Evaluation 2 of cytotoxicity]

Mesenchymal stem cells derived from the human adipose tissue (#C-12977 manufactured by Takara Bio Inc.) were suspended in mesenchymal stem cell growth medium 2 (C-28009 manufactured by Takara Bio Inc.) so as to be 8×10⁴ cells/1.8 mL, and seeded on a 6-well cell culture treatment plate (3516 manufactured by Corning Inc.). To the seeded mesenchymal stem cells derived from human adipose tissue was added 200 µL of the compound solution of each of Examples 1 to 8 diluted to 10-fold concentration with the mesenchymal stem cell growth medium 2, or mesenchymal stem cell growth medium alone (control), and after culturing for 24 hours, the culture solution was recovered. The activity of lactate dehydrogenase (LDH) contained in the recovered culture solution was measured by using LDH Cytotoxicity Detection Kit (MK401 manufactured by Takara Bio Inc.) as follows. To a freeze-dried product of Solution A was added 1 mL of purified water to completely dissolve therein, then, 250 µL of Solution A and 11.25 mL of Solution B were mixed to prepare Solution C. To 100 µL of the recovered culture solution was added 100 µL of Solution C, and the mixture was reacted at room temperature for 30 minutes. Absorbance at 490 nm was measured using Enspire (manufactured by PerkinElmer).

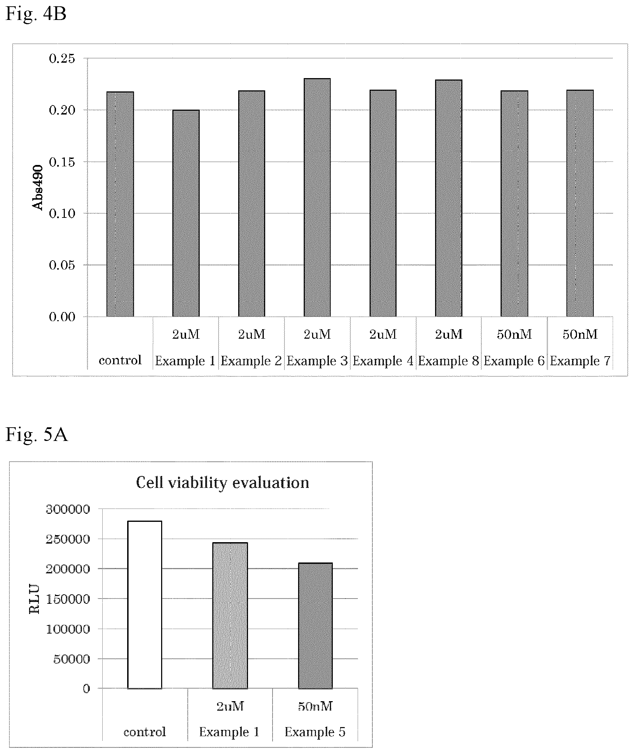

The results are shown in Table 3 and Table 4, and Fig. 4A and Fig. 4B.

**[Table 3]**

| | Concentration | Absorbance |
|---|---|---|
| Example 1 | 2 µM | 1.08 |
| Example 5 | 50 nM | 1.14 |

**[Table 4]**

| | Concentration | Absorbance |
|---|---|---|
| Example 1 | 2 µM | 0.73 |
| Example 2 | 2 µM | 1.02 |
| Example 3 | 2 µM | 1.02 |
| Example 4 | 2 µM | 1.20 |
| Example 8 | 2 µM | 1.18 |
| Example 6 | 50 nM | 1.02 |
| Example 7 | 50 nM | 1.02 |

### [Test Example 5: Evaluation 1 of cell proliferation activity]

Mesenchymal stem cells derived from the human adipose tissue (#C-12977 manufactured by Takara Bio Inc.) were suspended in mesenchymal stem cell growth medium 2 (C-28009 manufactured by Takara Bio Inc.) so as to be 2×10³ cells/90 µL, and seeded on a 96-well cell culture treatment plate (3585 manufactured by Corning Inc.). To the seeded mesenchymal stem cells derived from human adipose tissue was added 10 µL of the compound solution of each of Examples 1 to 8 diluted to 10-fold concentration with the mesenchymal stem cell growth medium 2, or mesenchymal stem cell growth medium alone (control), and the cells were cultured for 24 hours. Subsequently, the number of living cells was measured as follows using CellTiter-Glo (Registered Trademark) Luminescent Cell Viability Assay (G7570 manufactured by Promega). 100 µL well of a solution in which CellTiter-Glo (trademark) Substrate was dissolved in CellTiter-Glo (trademark) Buffer was added to each well of the above-mentioned 96-well cell culture treatment plate, and then, 100 µL thereof was transferred to 96-well cell culture treatment white plate (356701 manufactured by Corning Inc.), and the luminescence was measured with Enspire (manufactured by PerkinElmer).

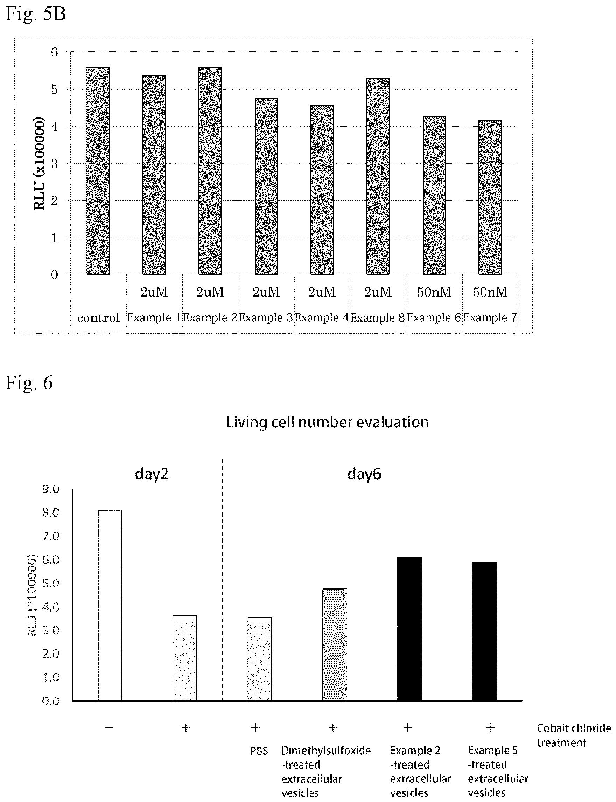

The results are shown in Table 5 and Table 6, and Fig. 5A and Fig. 5B.

**[Table 5]**

| | Concentration | Luminescence |
|---|---|---|
| Example 1 | 2 µM | 0.87 |
| Example 5 | 50 nM | 0.75 |

**[Table 6]**

| | Concentration | Luminescence |
|---|---|---|
| Example 1 | 2 µM | 0.96 |
| Example 2 | 2 µM | 1.00 |
| Example 3 | 2 µM | 0.82 |
| Example 4 | 2 µM | 0.85 |
| Example 8 | 2 µM | 0.95 |
| Example 6 | 50 nM | 0.76 |
| Example 7 | 50 nM | 0.74 |

### [Test Example 6: Pharmacological evaluation of extracellular vesicles (Pharmacological evaluation to cells under hypoxic treatment)]

### <Preparation of extracellular vesicles derived from human adipose tissue-derived mesenchymal cells>

Mesenchymal stem cells derived from the human adipose tissue (#C-12977 manufactured by Takara Bio Inc.) were suspended in mesenchymal stem cell growth medium 2 (C-28009 manufactured by Takara Bio Inc.) so as to be 4×10⁵ cells/10 mL, and seeded on a 100 mm cell culture surface-treated dish (#430167 manufactured by Corning Inc.) and cultured at 37°C under 5% CO₂ for 4 days. After 4 days, the cells were washed with PBS(-) (#166-23555 available from FUJIFILM Wako Pure Chemical Corporation), and then, the compounds of Examples 2 and 5 were each adjusted in a D-MEM (low glucose) (containing L-glutamine and phenol red) (#041-29775 available from FUJIFILM Wako Pure Chemical Corporation) medium so as to be a final concentration of 2 µM and 50 nM, respectively, and 10 mL thereof was added thereto and the cells were cultured for further 2 days. As a control, a material in which a D-MEM (low glucose) (containing L-glutamine and phenol red) medium containing the same concentration of dimethylsulfoxide had been prepared and added was also prepared. After 2 days, the respective culture supernatants were recovered. For purification of the extracellular vesicles, Capturem (trademark) Exosome Isolation Kit (Cell Culture) (#635723 manufactured by Takara Bio Inc.) was used. After the recovered culture supernatant was transferred into 50 mL of a centrifugal tube, the cells were centrifuged at 2,000×g for 10 minutes. Subsequently, the culture supernatant was transferred to Exosome Isolation Pre-Cleaning Column, and centrifuged at 1,000×g for 4 minutes. The culture supernatant after centrifugation was transferred to Capturem Maxiprep Exosome Isolation Column, and centrifuged at 1,000xg for 4 minutes. After centrifugation, Exosome Isolation Column was transferred to a new 50 mL of a centrifugal tube, 10 mL of Exosome Isolation Wash Buffer was added thereto and the mixture was centrifuged at 1,000xg for 2 minutes. Finally, Exosome Isolation Column was transferred to a new 50 mL of a centrifugal tube, 500 µL of Exosome Isolation Elution Buffer was added thereto and the mixture was centrifuged at 1,000xg for 2 minutes. The recovered extracellular vesicle solution was replaced with PBS(-) using Amicon Ultra-0.5, PLGC Ultracel-10 membrane, 10 kDa. For the detection of the extracellular vesicles contained in the recovered extracellular vesicle solution, PS Capture (trademark) exosome ELISA kit (anti-mouse IgG POD) (#297-79201 available from FUJIFILM Wako Pure Chemical Corporation) was used.

The results are shown in Table 7.

### <PS Capture (trademark) exosome ELISA kit>

To Reaction/Washing Buffer (1×) prepared by diluting Reaction/Washing Buffer (10×) 10-fold with purified water was added 1/100 amount of Exosome Binding Enhancer (100×) to prepare a Reaction/Washing Buffer (1×). Each extracellular vesicle solution was diluted 400-fold with the Reaction/Washing Buffer (1×). After washing Exosome Capture 96-well plate with 300 µL of the Reaction/Washing Buffer (1×) three times, 5-fold-diluted each extracellular vesicle solution, or the mesenchymal stem cell growth medium 2 in which no cell was cultured (negative control) was added to each well, and the mixture was reacted at room temperature for 2 hours while shaking with a microplate shaker. After completion of the reaction, the reaction mixture was discarded, and after washing each well with 300 µL of the Reaction/Washing Buffer (1×) three times, 100 µL of Primary Antibody Anti-CD63 (×100) diluted 1,000-fold with the Reaction/Washing Buffer (1×) was added thereto, and the mixture was reacted at room temperature for 1 hour while shaking with a microplate shaker. After completion of the reaction, the reaction mixture was discarded, and after washing each well with 300 µL of the Reaction/Washing Buffer (1×) three times, 100 µL of the Secondary Antibody HRP-conjugated Anti-mouse IgG (100×) diluted 1,000-fold with the Reaction/Washing Buffer (1×) was added thereto, and the mixture was reacted at room temperature for 1 hour while shaking with a microplate shaker. After completion of the reaction, the reaction mixture was discarded, and after washing each well with 300 µL of the Reaction/Washing Buffer (1×) five times, 100 µL of TMB Solution was added thereto, and after shaking with a microplate shaker for 1 minute, the mixture was reacted at room temperature for 30 minutes. After completion of the reaction, 100 µL of Stop Solution was added thereto, and after shaking with a microplate shaker for 5 seconds, the absorbance at 450 nm and 620 nm was measured with Enspire (manufactured by PerkinElmer). Based on the obtained measured values, a value (ΔAbs) obtained by subtracting the absorbance at 620 nm from the absorbance at 450 nm was calculated.

**[Table 7]**

| | Difference in absorbance |
|---|---|
| Dimethylsulfoxide-treated extracellular vesicles | 0.279 |
| Example 2-treated extracellular vesicles | 0.340 |
| Example 5-treated extracellular vesicles | 0.320 |

### <Evaluation of number of living cells>

H9C2 cells (#CRL-1446 available from ATCC) were suspended in a 10% exosome depleted FBS (#A2720803 available from Thermo Fisher Scientific K.K.)-containing D-MEM (high glucose) (containing L-glutamine, phenol red and sodium pyruvate) so as to be 50,000 cells/ mL, and seeded to a 96-well transparent flat-bottomed cell culture surface-treated plate (#3585 manufactured by Corning Inc.) with 100 µL/well, and the cells were cultured at 37°C under 5% CO₂ for 1 day. After one day, a 1M cobalt chloride solution (#036-03682 available from FUJIFILM Wako Pure Chemical Corporation) prepared with D-MEM (high glucose) (containing L-glutamine, phenol red and sodium pyruvate) was added with 50 µL/well so as to have a final concentration of 1 mM, and the cells were further cultured at 37°C under 5% CO₂ for 1 day (day 2). One day later, the medium was removed, 150 µL of a fresh medium, or a material in which 100 µL of a fresh medium and 50 µL of each extracellular vesicle solution prepared as mentioned above had been mixed was added thereto, and the cells were cultured for 4 days (day 6). Four days later, in order to measure the cell survival rate, 150 µL of an ATP reagent (CellTiter-Glo (Registered Trademark) Luminescent Cell Viability Assay manufactured by Promega) was added to each well and mixed, then, allowed to stand for 10 minutes. Ten minutes later, 100 µL of the mixture was transferred from each well to a plate for measurement, and the emission intensity (RLU value) was measured with Enspire (manufactured by Perkin Elmer). The number of the living cells was measured by subtracting the luminescence value of the medium alone from each measured value.

The results are shown in Table 8 and Fig. 6.

**[Table 8]**

| | Luminescence |
|---|---|
| PBS | 355875 |
| Dimethylsulfoxide-treated extracellular vesicles | 476870 |
| Example 2-treated extracellular vesicles | 609165 |
| Example 5-treated extracellular vesicles | 589585 |

### [Test Example 7: Evaluation 3 of promotion of secretion of extracellular vesicles]

### <Preparation of culture solution>

Human bone marrow-derived mesenchymal stem cells or human umbilical cord matrix-derived mesenchymal stem cells (#C-12974, #C-12971 manufactured by Takara Bio Inc.) were suspended in mesenchymal stem cell growth medium 2 (C-28009 manufactured by Takara Bio Inc.) so as to be 8×10⁴ cells/1.8 mL, and seeded on a 6-well cell culture treatment plate (3516 manufactured by Corning Inc.). To the seeded mesenchymal stem cells was added each 200 µL of the compounds of Examples 1 to 8 diluted to 10-fold concentration with the mesenchymal stem cell growth medium 2, and after culturing for 24 hours, the culture solution was recovered. Subsequently, the recovered culture solution was centrifuged under 20,000xg at 4°C for 60 minutes to prepare a culture solution from which cell fragments and Large Extracellular Vesicles were removed. For the detection of extracellular vesicles, PS Capture (trademark) exosome ELISA kit (anti-mouse IgG POD) (#297-79201 manufactured by FUJIFILM Wako Pure Chemical Corporation) was used.

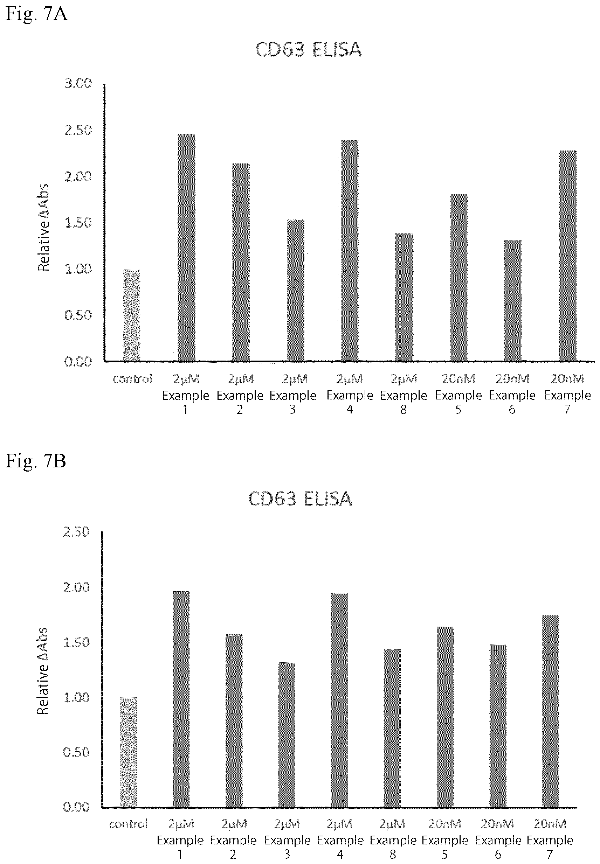

The results of the human bone marrow-derived mesenchymal stem cells are shown in Table 9 and Fig. 7A, and the results of the human umbilical cord matrix-derived mesenchymal stem cells are shown in Table 10 and Fig. 7B.

### <PS Capture (trademark) exosome ELISA kit>

To Reaction/Washing Buffer (1×) prepared by diluting Reaction/Washing Buffer (10×) 10-fold with purified water was added 1/100 amount of Exosome Binding Enhancer (100×) to prepare a Reaction/Washing Buffer (1×). Each culture solution was diluted 5-fold with the Reaction/Washing Buffer (1×). After washing Exosome Capture 96-well plate with 300 µL of the Reaction/Washing Buffer (1×) three times, 5-fold-diluted each extracellular vesicle solution, or the mesenchymal stem cell growth medium 2 in which no cell was cultured (negative control) was added to each well, and the mixture was reacted at room temperature for 2 hours while shaking with a microplate shaker. After completion of the reaction, the reaction mixture was discarded, and after washing each well with 300 µL of the Reaction/Washing Buffer (1×) three times, 100 µL of Primary Antibody Anti-CD63 (×100) diluted to 1,000-fold with the Reaction/Washing Buffer (1×) was added thereto, and the mixture were reacted at room temperature for 1 hour while shaking with a microplate shaker. After completion of the reaction, the reaction mixture was discarded, and after washing each well with 300 µL of the Reaction/Washing Buffer (1×) three times, 100 µL of Secondary Antibody HRP-conjugated Anti-mouse IgG (100×) diluted to 1,000-fold with the Reaction/Washing Buffer (1×) was added thereto, and the mixture were reacted at room temperature for 1 hour while shaking with a microplate shaker. After completion of the reaction, the reaction mixture was discarded, and after washing each well with 300 µL of the Reaction/Washing Buffer (1×) five times, 100 µL of TMB Solution was added, and after shaking with a microplate shaker for 1 minutes, the mixture was reacted at room temperature for 30 minutes. After completion of the reaction, 100 µL of Stop Solution was added thereto, and after shaking with a microplate shaker for 5 seconds, the absorbance at 450 nm and 620 nm was measured with Enspire (manufactured by PerkinElmer). Based on the obtained measured values, a value subtracting the absorbance at 620 nm from the absorbance at 450 nm was calculated and a relative value when the compound untreated group (control) was set to 1 was calculated.

**[Table 9]**

| | Concentration | Relative value of difference of absorbance |
|---|---|---|
| Example 1 | 2 µM | 2.46 |
| Example 2 | 2 µM | 2.14 |
| Example 3 | 2 µM | 1.53 |
| Example 4 | 2 µM | 2.39 |
| Example 8 | 2 µM | 1.39 |
| Example 5 | 20 nM | 1.81 |
| Example 6 | 20 nM | 1.31 |
| Example 7 | 20 nM | 2.88 |

**[Table 10]**

| | Concentration | Relative value of difference of absorbance |
|---|---|---|
| Example 1 | 2 µM | 1.97 |
| Example 2 | 2 µM | 1.57 |
| Example 3 | 2 µM | 1.31 |
| Example 4 | 2 µM | 1.95 |
| Example 8 | 2 µM | 1.44 |
| Example 5 | 20 nM | 1.65 |
| Example 6 | 20 nM | 1.48 |
| Example 7 | 20 nM | 1.74 |

### [Test Example 8: Evaluation 3 of cytotoxicity]

Mesenchymal stem cells derived from human bone marrow or mesenchymal stem cells derived from human umbilical cord matrix (#C-12974, #C-12971 manufactured by Takara Bio Inc.) were suspended in mesenchymal stem cell growth medium 2 (C-28009 manufactured by Takara Bio Inc.) so as to be 8×10⁴ cells/1.8 mL, and seeded on a 6-well cell culture treatment plate (3516 manufactured by Corning Inc.). To the seeded mesenchymal stem cells was added each 200 µL of the compounds of Examples 1 to 8 diluted to 10-fold concentration with the mesenchymal stem cell growth medium 2, and after culturing for 24 hours, the culture solution was recovered. The activity of lactate dehydrogenase (LDH) contained in the recovered culture solution was measured by using LDH Cytotoxicity Detection Kit (MK401 manufactured by Takara Bio Inc.). To a freeze-dried product of Solution A was added 1 mL of purified water to completely dissolve therein, then, 250 µL of Solution A and 11.25 mL of Solution B were mixed to prepare Solution C. To 100 µL of the recovered culture solution was added 100 µL of Solution C, and the mixture was reacted at room temperature for 30 minutes. Absorbance at 490 nm was measured using Enspire (manufactured by PerkinElmer). Based on the obtained measured values, a relative value when the compound untreated group (control) was set to 1 was calculated.

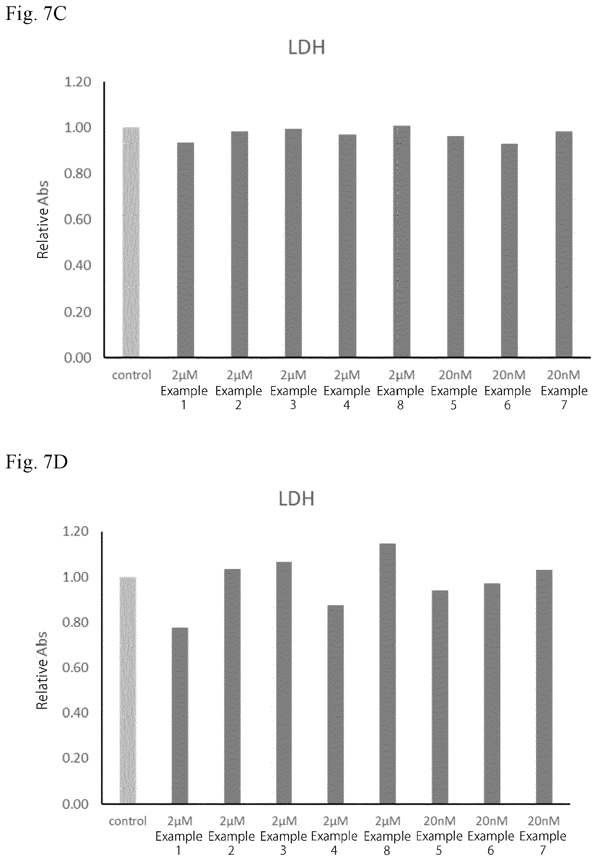

The results of the mesenchymal stem cells derived from human bone marrow are shown in Table 11 and Fig. 7C, and the results of the mesenchymal stem cells derived from human umbilical cord matrix are shown in Table 12 and Fig. 7D.

**[Table 11]**

| | Concentration | Relative value of absorbance |
|---|---|---|
| Example 1 | 2 µM | 0.93 |
| Example 2 | 2 µM | 0.99 |
| Example 3 | 2 µM | 0.99 |
| Example 4 | 2 µM | 0.97 |
| Example 8 | 2 µM | 1.01 |
| Example 5 | 20 nM | 0.96 |
| Example 6 | 20 nM | 0.93 |
| Example 7 | 20 nM | 0.99 |

**[Table 12]**

| | Concentration | Relative value of absorbance |
|---|---|---|
| Example 1 | 2 µM | 0.78 |
| Example 2 | 2 µM | 1.04 |
| Example 3 | 2 µM | 1.06 |
| Example 4 | 2 µM | 0.88 |
| Example 8 | 2 µM | 1.15 |
| Example 5 | 20 nM | 0.94 |
| Example 6 | 20 nM | 0.97 |
| Example 7 | 20 nM | 1.03 |

### [Test Example 9: Evaluation 2 of cell proliferation activity]

Mesenchymal stem cells derived from human bone marrow or mesenchymal stem cells derived from human umbilical cord matrix (#C-12974, #C-12971 manufactured by Takara Bio Inc.) were suspended in mesenchymal stem cell growth medium 2 (C-28009 manufactured by Takara Bio Inc.) so as to be 2×10³ cells/90 µL, and seeded on a 96-well cell culture treatment plate (3585 manufactured by Corning Inc.). To the seeded mesenchymal stem cells was added each 10 µL of the compounds of Examples 1 to 8 diluted to 10-fold concentration with the mesenchymal stem cell growth medium 2, and the cells were cultured for 24 hours. Next, according to the following method, the number of living cells was measured using CellTiter-Glo (Registered Trademark) Luminescent Cell Viability Assay (G7570 manufactured by Promega).

CellTiter-Glo (Registered Trademark) Substrate was dissolved in CellTiter-Glo (Registered Trademark) Buffer. The prepared solution was added to each well with 100 pL/well, then, 100 µL thereof was apportioned to a 96-well cell culture treatment white plate (356701 manufactured by Corning Inc.), and luminescence was measured with Enspire (manufactured by PerkinElmer). A relative value when the compound untreated group (control) was set to 1 was calculated.

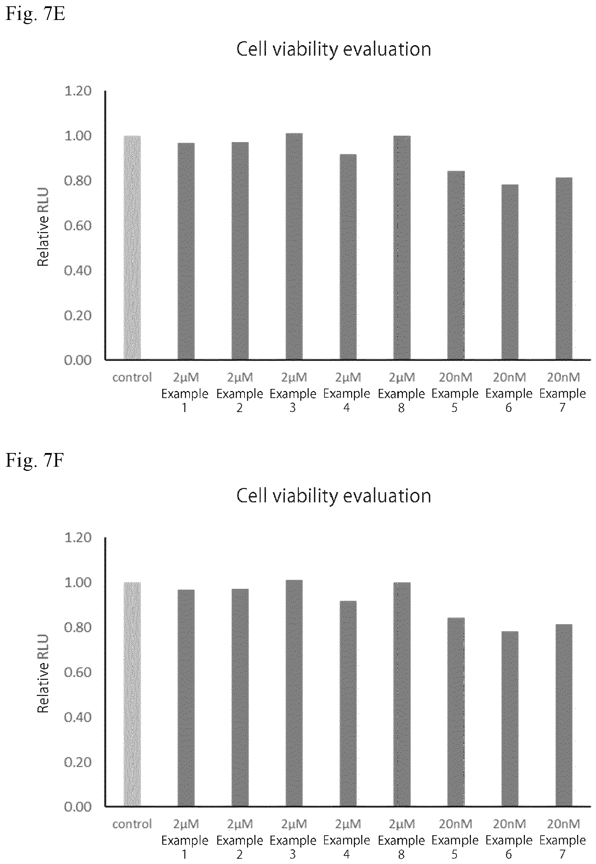

The results of the mesenchymal stem cells derived from human bone marrow are shown in Table 13 and Fig. 7E, and the results of the mesenchymal stem cells derived from human umbilical cord matrix are shown in Table 14 and Fig. 7F.

**[Table 13]**

| | Concentration | Relative value of luminescence |
|---|---|---|
| Example 1 | 2 µM | 0.91 |
| Example 2 | 2 µM | 0.96 |
| Example 3 | 2 µM | 1.00 |
| Example 4 | 2 µM | 0.86 |
| Example 8 | 2 µM | 0.93 |
| Example 5 | 20 nM | 0.96 |
| Example 6 | 20 nM | 0.92 |
| Example 7 | 20 nM | 0.82 |

**[Table 14]**

| | Concentration | Relative value of luminescence |
|---|---|---|
| Example 1 | 2 µM | 0.97 |
| Example 2 | 2 µM | 0.97 |
| Example 3 | 2 µM | 1.01 |
| Example 4 | 2 µM | 0.92 |
| Example 8 | 2 µM | 1.00 |
| Example 5 | 20 nM | 0.84 |
| Example 6 | 20 nM | 0.78 |
| Example 7 | 20 nM | 0.82 |

### [Test Example 10: Evaluation 4 of promotion of secretion of extracellular vesicles]

### <Preparation of culture supernatant>

After stirring 10 mL of deactivated FBS and 2 mL of a 50 % (w/v%) Poly(ethylene glycol) 10,000 solution (#81280 available from Sigma-Aldrich Co., LLC) at 4°C for 2 hours, extracellular vesicles were precipitated under the centrifugation conditions of 1,500×g at 4°C for 30 minutes, and the supernatant was recovered to prepare Exosome-free FBS. Next, 293 cells (CRL-1573TM available from ATCC) or 293T cells (CRL-3216TM available from ATCC) were suspended in Advanced DMEM (#12491015 available from Thermo Fisher Scientific K.K.) containing 2% (v/v%) Exosome-free FBS with 2×10⁴ cells/200 µL, seeded on an adhesive surface 96-well plate (#3595 manufactured by Corning Inc.) and pre-culture was carried out for 24 hours. After 24 hours, the culture solution was removed, a mixed solution of 199.5 µL of 2% (v/v%) Exosome-free FBS-containing Advanced DMEM and 0.5 µL (final concentration: 0 to 1 µM) of the compound of Example 1, 5 or 9 prepared in DMSO was added thereto, and the cells were cultured for 24 hours. Thereafter, the culture solution was recovered in a 1.5 mL tube, the cells were precipitated under centrifugation conditions of 300xg at 4°C for 5 minutes, and the culture supernatant was recovered. Subsequently, the recovered culture supernatant was subjected to centrifugation under the conditions of 2,000xg at 4°C for 20 minutes to precipitate cell fragments, and the supernatant was recovered. Further, the recovered supernatant was subjected to centrifugation under the conditions of 10,000xg at 4°C for 30 minutes, and the supernatant was recovered. For detection of the extracellular vesicles contained in the supernatant after centrifugation of 10,000xg, Tim4-human CD63 ELISA method and Tim4-human CD81ELISA method mentioned later was used.

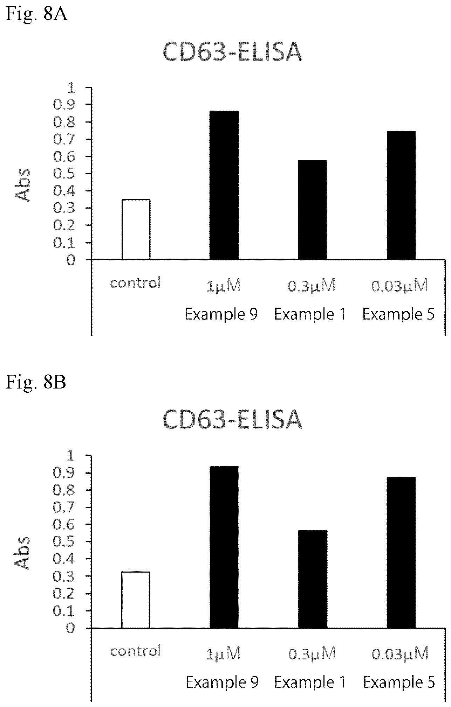

The results of the 293 cells are shown in Fig. 8A, and the results of the 293T cells are shown in Fig. 8B.

### <Tim4-human CD63 ELISA method>

100 µL/well of 1 µg/mL Tim4 protein prepared in Carbonate Buffer (solution containing 71.4 mM NaHC03 and 28.6 mM Na₂CO₃) was added to a 96-well plate (#3801-096 manufactured by IWAKI & CO., LTD.) and made it a solid phase. After washing the 96-well plate with a TBST solution, 200 µL/well of the TBST solution containing 1% (w/v%) BSA was added thereto and a blocking treatment for 1 hour was carried out. Next, 90 µL of the supernatant recovered as mentioned above was added to the well, and 10 µL of a 20 mM CaCl₂ solution was further added to bind the extracellular vesicles to the Tim4 protein. Subsequently, 100 µL/well of 1 µg/mL Anti-human CD63 Antibody (#353014 available from BioLegend Inc.) diluted with the TBST solution containing 2 mM CaCl₂ was added thereto and the reaction was carried out for 1 hour. 200 µL/well of the TBST solution containing 2 mM CaCl₂ was added thereto, and thereafter, the TBST solution was removed. After repeating this operation three times, 100 µL/well of 80 ng/mL HRP-conjugated Anti-mouse IgG (#405306 available from BioLegend Inc.) diluted with the TBST solution containing 2 mM CaCl₂ was added thereto and the reaction was carried out for 1 hour. Finally, 100 µL/well of the TMB solution (#05298-80 available from Nacalai Tesque, Inc.) was added thereto, the reaction was carried out for 30 minutes, then, 100 µL/well of 1M H₂SO₄ solution was added to stop the reaction, and luminescence at 450 nm was measured to determine the amount of the extracellular vesicles.

### <Tim4-human CD81ELISA method>

The Tim4-human CD81 ELISA method was carried out in the same method as in the above-mentioned Tim4-human CD63 ELISA method except for replacing 1 µg/ mL Anti-human CD63 Antibody with 0.5 µg/ mL Anti-human CD81 Antibody (#349502 available from BioLegend Inc.).

### [Test Example 11: Evaluation 5 of promotion of secretion of extracellular vesicles]

After stirring 10 mL of deactivated FBS and 2 mL of a 50 % (w/v%) Poly(ethylene glycol) 10,000 solution (#81280 available from Sigma-Aldrich Co., LLC) at 4°C for 2 hours, extracellular vesicles were precipitated under the centrifugation conditions of 1,500×g at 4°C for 30 minutes, and the supernatant was recovered to prepare Exosome-free FBS. Next, 293 cells (CRL-1573TM available from ATCC) or 293T cells (CRL-3216TM available from ATCC) were suspended in Advanced DMEM (#12491015 available from Thermo Fisher Scientific K.K.) containing 2% (v/v%) Exosome-free FBS with 2×10⁵ cells/1,000 µL, seeded on an adhesive surface 12-well plate (#3513 manufactured by Corning Inc.), and pre-culture for 24 hours was carried out. After 24 hours, the culture solution was removed, a mixed solution of 999 µL of Advanced DMEM containing 2% (v/v%) Exosome-free FBS and 1 µL (final concentration: 0 to 1 µM) of the compound of Example 1, 5 or 9 prepared in DMSO was added thereto, and the cells were cultured for 24 hours. Thereafter, the culture solution was recovered in a 1.5 mL tube, the cells were precipitated under the centrifugation conditions of 300×g at 4°C for 5 minutes, and the culture supernatant was recovered. Subsequently, the recovered culture supernatant was subjected to centrifugation under the conditions of 2,000×g at 4°C for 20 minutes to precipitate cell fragments, and the supernatant was recovered. Further, the recovered supernatant was subjected to centrifugation under the conditions of 10,000×g at 4°C for 30 minutes, and the supernatant was recovered. An amount of the extracellular vesicles contained in 500 µL of the supernatant after centrifugation of 10,000×g was determined using a nanoparticle tracking analysis system (#NanoSIGHT LM10 manufactured by Malvern Panalytical).

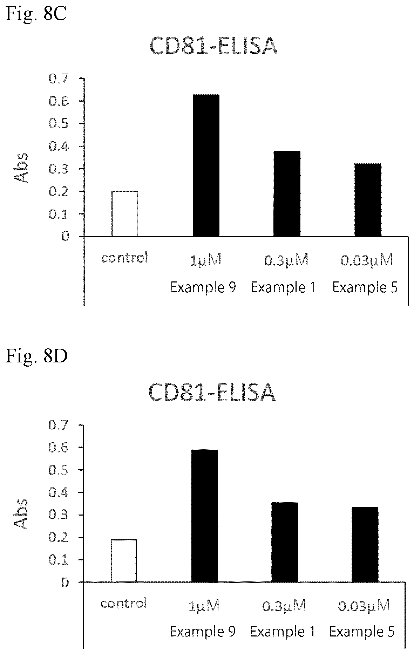

The results of the 293 cells are shown in Fig. 8C, and the results of the 293T cells are shown in Fig. 8D.

### [Test Example 12: Evaluation 4 of cytotoxicity]

After stirring 10 mL of deactivated FBS and 2 mL of a 50 % (w/v%) Poly(ethylene glycol) 10,000 solution (#81280 available from Sigma-Aldrich Co., LLC) at 4°C for 2 hours, extracellular vesicles were precipitated under the centrifugation conditions of 1,500×g at 4°C for 30 minutes, and the supernatant was recovered to prepare Exosome-free FBS. Next, 293 cells (CRL-1573TM available from ATCC) or 293T cells (CRL-3216TM available from ATCC) were suspended in Advanced DMEM (#12491015 available from Thermo Fisher Scientific K.K.) containing 2% (v/v%) Exosome-free FBS with 2×10⁴ cells/200 µL, seeded on an adhesive surface 96-well plate (#3595 manufactured by Corning Inc.), and pre-culture for 24 hours was carried out. After 24 hours, the culture solution was removed, a mixed solution of 199.5 µL of Advanced DMEM containing 2% (v/v%) Exosome-free FBS and 0.5 µL (final concentration: 0 to 1 µM) of the compound of Example 1, 5 or 9 prepared in DMSO was added thereto, and the cells were cultured for 24 hours. Thereafter, the plate was subjected to centrifugation under the conditions of 1,200×g at 4°C for 1 hour to precipitate the cells, and 50 µL of the culture supernatant was transferred to a 96-well microplate (#195-96F manufactured by WATSON). The activity of lactate dehydrogenase (LDH) contained in the culture supernatant was measured using Cytotoxicity LDH Assay Kit-WST (#CK12 manufactured by Dojindo Laboratories). 5 mL of Assay Buffer was added to 500 tests of Dye Mixture and dissolved to prepare Working Solution. To the 96-well microplate to which 50 µL of the culture supernatant was transferred was added 50 µL of the Working Solution, and after mixing well, a coloring reaction was carried out at room temperature for 30 minutes. The reaction was stopped by adding 25 µL of Stop Solution, and the cytotoxicity was evaluated by measuring the absorbance at 490 nm. The activities of LDH of the dead cell group (sample to which 20 µL of Lysis Buffer was added to compound-untreated cells 15 minutes before recovery of the culture solution) and the culture solution (culture solution in which no cell culture was carried out) were also measured in the same manner, and cytotoxicity of each compound was evaluated.

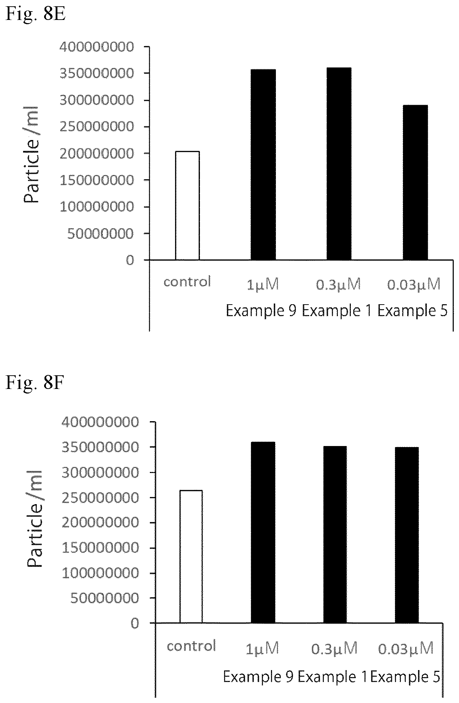

The results of the 293 cells are shown in Fig. 8E, and the results of the 293T cells are shown in Fig. 8F.

### [Test Example 13: Evaluation 3 of cell proliferation activity]

After stirring 10 mL of deactivated FBS and 2 mL of a 50% (w/v%) Poly(ethylene glycol) 10,000 solution (#81280 available from Sigma-Aldrich Co., LLC) at 4°C for 2 hours, extracellular vesicles were precipitated under the centrifugation conditions of 1,500×g at 4°C for 30 minutes, and the supernatant was recovered to prepare Exosome-free FBS. Next, 293 cells (CRL-1573TM available from ATCC) or 293T cells (CRL-3216TM available from ATCC) were suspended in Advanced DMEM (#12491015 available from Thermo Fisher Scientific K.K.) containing 2% (v/v%) Exosome-free FBS with 2×10⁴ cells/200 µL, seeded on an adhesive surface 96-well plate (#3595 manufactured by Corning Inc.), and pre-culture for 24 hours was carried out. After 24 hours, the culture solution was removed, a mixed solution of 199.5 µL of Advanced DMEM containing 2% (v/v%) Exosome-free FBS and 0.5 µL (final concentration: 0 to 1 µM) of the compound of Example 1, 5 or 9 prepared in DMSO was added thereto, and the cells were cultured for 24 hours. After 24 hours, the plate was subjected to centrifugation under the conditions of 1,200×g at 4°C for 1 hour to precipitate the cells and the culture supernatant was removed, and a mixed solution of 90 µL of Advanced DMEM and 10 µL of Cell Count Reagent SF for measurement of a number of living cells (#07553 available from Nacalai Tesque, Inc.) was added thereto, and the cells were cultured for 30 minutes. After 30 minutes, absorbance at 450 nm was measured to evaluate cell proliferation activity.

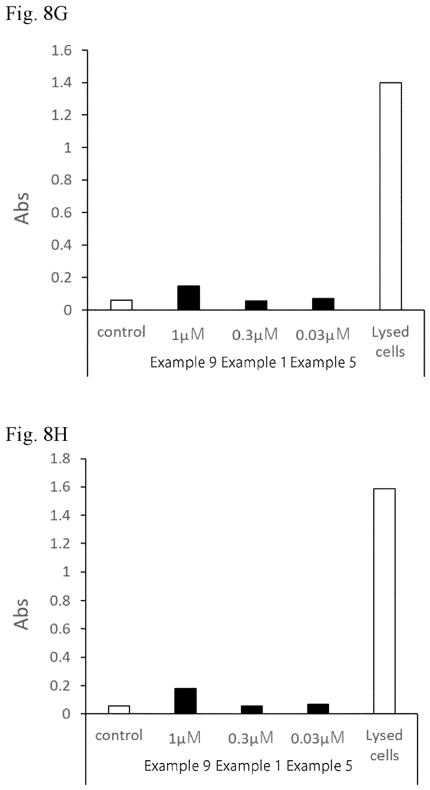

The results of the 293 cells are shown in Fig. 8G, and the results of the 293T cells are shown in Fig. 8H.

### [Results]

From the results of Test Example 1, it can be understood that the compound of Example 1 or 5 promotes secretion of CD9 and/or CD63-positive extracellular vesicles from U-87 MG cells. In particular, it can be considered that the compound of Example 1 strongly promoted secretion of CD9 and/or CD63-positive extracellular vesicles, and the compound of Example 5 strongly promoted secretion of CD63-positive extracellular vesicles.

From the results of Test Example 2, remarkable increase in the LDH amount in the culture supernatant was not observed in the compound of Example 1 with a concentration of at least about 2 µM or less and in the compound of Example 5 with a concentration of at least about 50 nM or less, so that it could be considered that cytotoxicity to U-87 MG cells was low.

Accordingly, the compound (or the composition containing the same) of Example 1 or 5 promotes secretion of extracellular vesicles from U-87 MG cells. Further, the compound of Examples 1 or 5 (or the composition containing the same) promotes secretion of extracellular vesicles from U-87 MG cells whereas they have low cytotoxicity to U-87 MG cells.

From the results of Test Examples 3 and 7, it can be understood that the compounds of Examples 1 to 8 promoted an amount of secretion of CD63-positive extracellular vesicles from mesenchymal stem cells derived from human adipose tissue, mesenchymal stem cells derived from human bone marrow and mesenchymal stem cells derived from human umbilical cord matrix.

From the results of Test Examples 4 and 8, remarkable increase in the LDH amount in the culture supernatant was not observed in the compounds of Examples 1 to 4 and 8 with a concentration of at least about 2 µM or less and in the compounds of Examples 5 to 7 with a concentration of at least about 50 nM or less, so that it could be understood that they have low cytotoxicity to mesenchymal stem cells derived from human adipose tissue, mesenchymal stem cells derived from human bone marrow and mesenchymal stem cells derived from human umbilical cord matrix.

From the results of Test Examples 5 and 9, it can be understood that it does not have a significant effect on the proliferative activity of mesenchymal stem cells derived from human adipose tissue, mesenchymal stem cells derived from human bone marrow and mesenchymal stem cells derived from human umbilical cord matrix in the compounds of Examples 1 to 4 and 8 with a concentration of at least about 2 µM or less and in the compounds of Examples 5 to 7 with a concentration of at least about 50 nM or less.

Accordingly, the compounds of Examples 1 to 8 (or the composition containing the same) promote secretion of extracellular vesicles from mesenchymal stem cells derived from human adipose tissue, mesenchymal stem cells derived from human bone marrow and mesenchymal stem cells derived from human umbilical cord matrix. Alternatively, the compounds of Examples 1 to 8 (or the composition containing the same) promote secretion of extracellular vesicles from mesenchymal stem cells derived from human adipose tissue, mesenchymal stem cells derived from human bone marrow and mesenchymal stem cells derived from human umbilical cord matrix even though they have low cytotoxicities to mesenchymal stem cells derived from human adipose tissue, mesenchymal stem cells derived from human bone marrow and mesenchymal stem cells derived from human umbilical cord matrix. Alternatively, the compounds of Examples 1 to 8 (or the composition containing the same) promote secretion of extracellular vesicles from mesenchymal stem cells derived from human adipose tissue, mesenchymal stem cells derived from human bone marrow and mesenchymal stem cells derived from human umbilical cord matrix even though they do not have significant effects on proliferation of mesenchymal stem cells derived from human adipose tissue, mesenchymal stem cells derived from human bone marrow and mesenchymal stem cells derived from human umbilical cord matrix. Alternatively, the compounds of Examples 1 to 8 (or the composition containing the same) promote secretion of extracellular vesicles from mesenchymal stem cells derived from human adipose tissue, mesenchymal stem cells derived from human bone marrow and mesenchymal stem cells derived from human umbilical cord matrix even though they have low cytotoxicities to mesenchymal stem cells derived from human adipose tissue, mesenchymal stem cells derived from human bone marrow and mesenchymal stem cells derived from human umbilical cord matrix and they do not have significant effects on proliferation of mesenchymal stem cells derived from human adipose tissue, mesenchymal stem cells derived from human bone marrow and mesenchymal stem cells derived from human umbilical cord matrix.

From the results of Test Example 6, higher absorbance was obtained from extracellular vesicles obtained from mesenchymal stem cells derived from human adipose tissue treated with the compound of Example 2 or 5 as compared with extracellular vesicles obtained from mesenchymal stem cells derived from human adipose tissue treated with dimethylsulfoxide. Further, it can be understood that the number of living cells of H9C2 cells under cobalt chloride treatment (that is, under hypoxia treatment) is higher in the group in which extracellular vesicles obtained from mesenchymal stem cells derived from human adipose tissue treated with the compound of Example 2 or 5 as compared with the extracellular vesicles untreated group or the group in which extracellular vesicles obtained from mesenchymal stem cells derived from human adipose tissue treated with dimethylsulfoxide.

Accordingly, it can be considered that extracellular vesicles obtained from mesenchymal stem cells derived from human adipose tissue treated with the compound of Example 2 or 5 (or the composition containing the same) have a cytoprotective action against H9C2 cells under hypoxia treatment.

From the results of Test Examples 10 and 11, it can be understood that the compound of Example 1, 5 or 9 promotes an amount of secretion of CD63 and/or CD81-positive extracellular vesicles from 293 cells and 293T cells.

From the results of Test Example 12, remarkable increase in the LDH amount in the culture supernatant was not observed in the compound of Example 1 with a concentration of at least about 0.3 µM or less, the compound of Example 5 with a concentration of at least about 0.03 µM (30 nM) or less, and the compound of Example 9 with a concentration of at least about 1 µM or less, so that it could be considered that they have low cytotoxicity to 293 cells and 293T cells.

From the results of Test Example 13, it can be understood that they do not significantly affect the proliferative activity of 293 cells and 293T cells in the compound of Example 1 with a concentration of at least about 0.3 µM or less, the compound of Example 5 with a concentration of at least about 0.03 µM (30 nM) or less, and the compound of Example 9 with a concentration of at least about 1 µM or less.

Accordingly, the compound of Example 1, 5 or 9 (or the composition containing the same) promotes secretion of extracellular vesicles from 293 cells and 293T cells. Alternatively, the compound of Example 1, 5 or 9 (or the composition containing the same) promotes secretion of extracellular vesicles from 293 cells and 293T even though they have low cytotoxicity to 293 cells and 293T cells. Alternatively, the compound of Example 1, 5 or 9 (or the composition containing the same) promotes secretion of extracellular vesicles from 293 cells and 293T cells even though they do not have a significant effect on the proliferation of 293 cells and 293T cells. Alternatively, the compound of Example 1, 5 or 9 (or the composition containing the same) promotes secretion of extracellular vesicles from 293 cells and 293T cells even though they have low cytotoxicity to 293 cells and 293T cells and they do not have a significant effect on 293 cells and 293T cells.

As mentioned above, from the results of Test Examples 1, 3, 7, 10 and 11, it can be understood that the compounds of Examples 1 to 9 can promote secretion of extracellular vesicles derived from various cells (preferably extracellular vesicles derived from mesenchymal stem cells). Accordingly, the composition (or the compound) described in the present specification can promote secretion of extracellular vesicles derived from various cells (preferably extracellular vesicles derived from mesenchymal stem cells).

Further, from the results of Test Examples 2, 4, 8 and 12, remarkable increase in the LDH amount in the culture supernatant was not observed in the compounds of Examples 1 to 9. Accordingly, the composition (or the compound) described in the present specification has low cytotoxicity to various cells (preferably cytotoxicity to mesenchymal stem cells), and is safe.

Moreover, from the results of Test Examples 5, 9 and 13, the compounds of Examples 1 to 9 did not have a significant effect on the cell proliferation activity to various cells (preferably cell proliferation activity to mesenchymal stem cells). Accordingly, the composition (or the compound) described in the present specification did not have a significant effect on cell proliferation activity to various cells (preferably cell proliferation activity to mesenchymal stem cells), and is safe.

Furthermore, from the results of Test Example 6, extracellular vesicles (preferably exosome) obtained from mesenchymal stem cells (preferably mesenchymal stem cells derived from human adipose tissue) treated with the compound of Example 2 or 5 increased the number of living cells of the cells under stress such as under hypoxia treatment, etc., (for example, cells derived from the heart). Accordingly, it can be considered that extracellular vesicles (preferably exosome) obtained from the cells treated with the composition (or the compound) described in the present specification (preferably mesenchymal stem cells) have a cytoprotective effect on cells under stress such as hypoxia treatment, etc.

## Claims

1. A composition for promoting secretion of extracellular vesicles, which comprises a compound having a structure represented by the formula I: 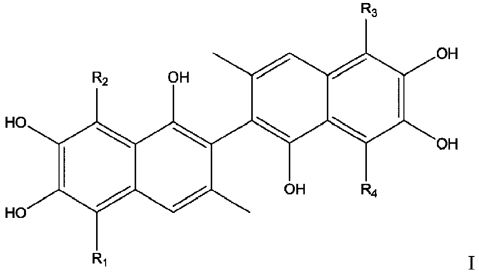 wherein
R₁ is C₁-C₆ alkyl which may be substituted by hydroxy, halogen, amino, nitro or cyano, or -C(O)-R₇,
R₂ is hydrogen, hydroxy, C₁-C₆ alkyl or -C(O)-R₅,
R₃ is C₁-C₆ alkyl which may be substituted by hydroxy, halogen, amino, nitro or cyano, or -C(O)-R₈,
R₄ is hydrogen, hydroxy, C₁-C₆ alkyl or -C(O)-R₆,
R₅ and R₆ are independently hydrogen; hydroxy; C₁-C₆ alkyl which may be substituted by hydroxy, halogen, amino, nitro or cyano; or C₁-C₆ alkoxy which may be substituted by hydroxy, halogen, amino, nitro or cyano,
R₇ and R₈ are independently hydrogen, hydroxy or -NR₉R₁₀,
R₉ and R₁₀ are independently hydrogen, or C₁-C₆ alkyl which may be substituted by hydroxy, halogen, amino, nitro, cyano or phenyl,
a racemate thereof or a stereoisomer thereof, or a salt thereof.

2. A composition for promoting secretion of extracellular vesicles, which comprises a compound having a structure of the formula II:
A-B II
wherein
A is 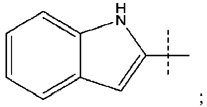 or 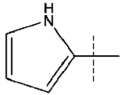
B is 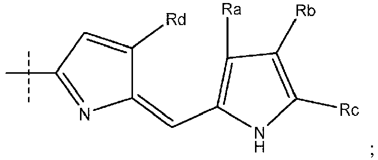 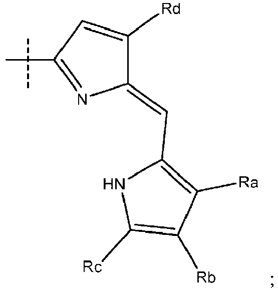 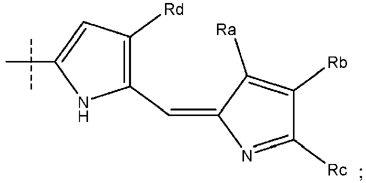 or 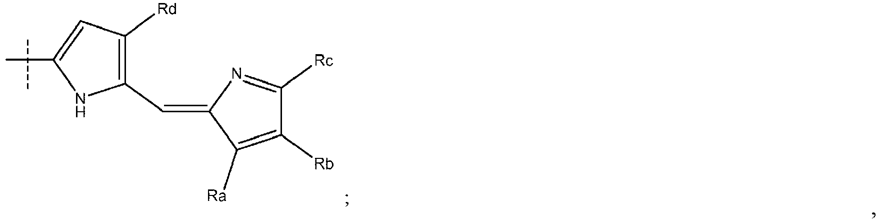 in these formulae,
each Ra to Rc are independently hydrogen, hydroxy, halogen, or C₁-C₆ alkyl which may be substituted by C₁-C₆ alkyl, hydroxy, halogen, amino, nitro or cyano,
each Rd are independently hydrogen, hydroxy, C₁-C₆ alkyl, or C₁-C₆ alkoxy which may be substituted by hydroxy, halogen, amino, nitro or cyano, or a stereoisomer thereof, or a salt thereof.

3. The composition according to Claim 1, wherein R₁ is C₁-C₆ alkyl and R₃ is C₁-C₆ alkyl.

4. The composition according to Claim 1 or 3, wherein R₂ is -C(O)-R₅ and R₄ is -C(O)-R₆.

5. The composition according to Claim 4, wherein R₅ is hydrogen and R₆ is hydrogen.

6. The composition according to Claim 1, wherein the compound having a structure of the formula I is selected from the group consisting of the following:

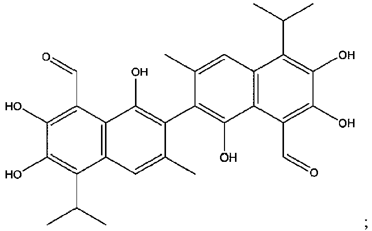

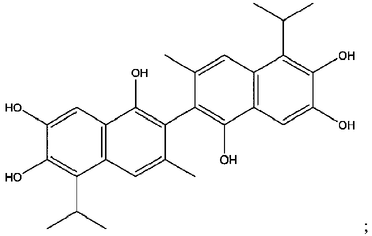

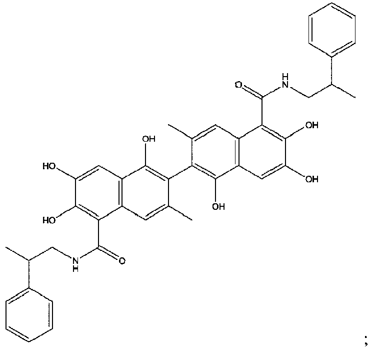

7. The composition according to Claim 1, wherein the compound having a structure of the formula I is selected from the group consisting of (±)-gossypol, (S)-gossypol, (+)-apogossypol, (R)-(-)-gossypol and sabutoclax.

8. The composition according to Claim 2, wherein A is the following:

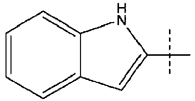

9. The composition according to Claim 2 or 8, wherein B is the following: 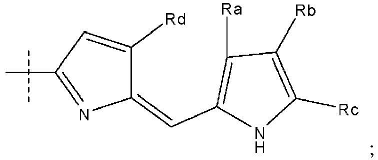 or

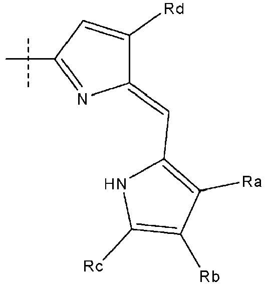

10. The composition according to any one of Claims 2, 8 and 9, wherein Rd is C₁-C₆ alkoxy.

11. The composition according to Claim 2, wherein the compound having a structure of the formula II is selected from the group consisting of the following: 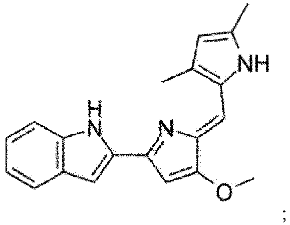 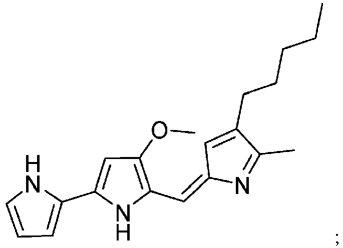 and

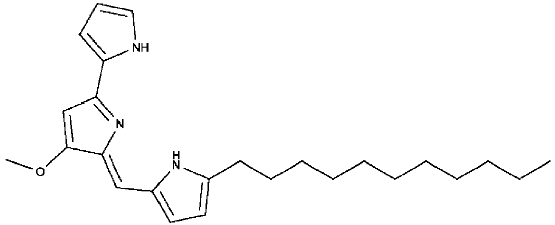

12. The composition according to Claim 2, wherein the compound having a structure of the formula II is selected from the group consisting of obatoclax, prodigiosin and undecylprodigiosin.

13. The composition according to any one of Claims 1 to 12, which is for promoting secretion of extracellular vesicles from cells derived from adipose tissues.

14. The composition according to any one of Claims 1 to 13, which is for promoting secretion of extracellular vesicles from mesenchymal stem cells.

15. The composition according to any one of Claims 1 to 14, wherein it is a medium composition.

16. A method for promoting secretion of extracellular vesicles from cells *in vitro* or *ex vivo,* which comprises using the composition according to Claim 15.

17. A culture supernatant obtained by the method according to Claim 16.

18. Extracellular vesicles obtained from the culture supernatant according to Claim 17.

19. A method for producing extracellular vesicles *in vitro* or *ex vivo* using the composition according to any one of Claims 1 to 15.

20. The method according to Claim 19, which comprises a step of bringing the composition according to any one of Claims 1 to 15 into contact with cells.
